(19) Europäisches Patentamt
European Patent Office
Office européen des brevets

(11) Veröffentlichungsnummer: **0 469 460 B1**

(12) **EUROPÄISCHE PATENTSCHRIFT**

(45) Veröffentlichungstag der Patentschrift: **18.10.95**

(51) Int. Cl.[6]: **C07D 251/16**, C07D 251/18, C07D 251/52, A01N 47/36

(21) Anmeldenummer: **91112468.3**

(22) Anmeldetag: **25.07.91**

Die Akte enthält technische Angaben, die nach dem Eingang der Anmeldung eingereicht wurden und die nicht in dieser Patentschrift enthalten sind.

# (54) Herbizide Sulfonylharnstoffe, Verfahren und Zwischenprodukte zu ihrer Herstellung und ihre Verwendung.

(30) Priorität: **03.08.90 DE 4024754**

(43) Veröffentlichungstag der Anmeldung:
**05.02.92 Patentblatt 92/06**

(45) Bekanntmachung des Hinweises auf die Patenterteilung:
**18.10.95 Patentblatt 95/42**

(84) Benannte Vertragsstaaten:
**AT BE CH DE ES FR GB IT LI NL**

(56) Entgegenhaltungen:
**EP-A- 0 084 020**
**EP-A- 0 103 543**
**EP-A- 0 169 815**
**EP-A- 0 336 587**
**EP-B- 0 070 804**
**FR-A- 2 590 570**

**CHEMICAL ABSTRACTS, Band 112, Nr. 9, 26. Februar 1990, Columbus, Ohio, USA MORISHIGE, JIRO et al. "Synergistic herbicides containing a triazine and an acetanilide or a dinitroaniline "**

(73) Patentinhaber: **BASF Aktiengesellschaft**
**Carl-Bosch-Strasse 38**
**D-67063 Ludwigshafen (DE)**

(72) Erfinder: **Hamprecht, Gerhard, Dr.**
**Rote-Turm-Strasse 28**
**W-6940 Weinheim (DE)**
Erfinder: **Mayer, Horst, Dr.**
**Faselwiese 19**
**W-6700 Ludwigshafen (DE)**
Erfinder: **Westphalen, Karl-Otto, Dr.**
**Mausbergweg 58**
**W-6720 Speyer (DE)**
Erfinder: **Walter, Helmut, Dr.**
**Gruenstadter Strasse 82**
**W-6719 Obrigheim (DE)**
Erfinder: **Gerber, Matthias, Dr.**
**Ritterstrasse 3**
**W-6704 Mutterstadt (DE)**
Erfinder: **Grossmann, Klaus, Dr.**
**Wilhelm-Busch-Strasse 5**
**W-6703 Limburgerhof (DE)**
Erfinder: **Rademacher, Wilhelm, Dr.**
**Austrasse 1**
**W-6703 Limburgerhof (DE)**

**EP 0 469 460 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft substituierte Sulfonylharnstoffe der allgemeinen Formel I,

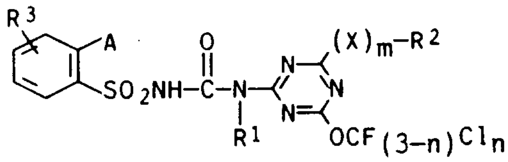

I,

in der n und m für 0 oder 1 stehen und die Substituenten folgende Bedeutung haben:

$R^1$

Wasserstoff, $C_1$-$C_4$-Alkyl, $C_3$-$C_6$-Alkenyl oder $C_3$-$C_6$-Alkinyl;

$R^2$

Halogen oder Trifluormethyl, wenn m für 0 steht oder $C_1$-$C_4$-Alkyl, $C_3$-$C_6$-Alkenyl oder $C_3$-$C_6$-Alkinyl, wenn m 1 bedeutet oder Trifluor- oder Chlordifluormethyl wenn X für 0 oder S und m für 1 steht;

X

O, S oder N-$R^4$, wobei $R^4$ für Wasserstoff oder $C_1$-$C_4$-Alkyl steht;

$R^3$

Wasserstoff, Halogen, $C_1$-$C_4$-Alkyl, $C_1$-$C_4$-Halogenalkyl oder $C_1$-$C_4$-Alkoxy;

A

$C_1$-$C_4$-Halogenalkyl, ein Halogenatom, Cyano, Nitro, $C_1$-$C_4$-Alkoxy, $C_1$-$C_4$-Alkylthio, $C_1$-$C_4$-Alkylsulfinyl- oder sulfonyl oder ein Rest

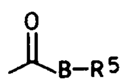

wobei

B

ein Sauerstoffatom oder eine Alkyliminogruppe N-$R^6$;

$R^5$

Wasserstoff, eine $C_1$-$C_6$-Alkylgruppe, welche bis zu drei der folgenden Reste tragen kann: Halogen, $C_1$-$C_4$-Alkoxy, $C_1$-$C_4$-Alkylthio, $C_1$-$C_4$-Halogenalkoxy, $C_2$-$C_4$-Alkoxy-$C_1$-$C_2$-alkoxy, $C_3$-$C_7$-Cycloalkyl und/oder Phenyl; eine $C_5$-$C_7$-Cycloalkylgruppe, welche bis zu drei $C_1$-$C_4$-Alkylgruppen tragen kann; eine $C_3$-$C_6$-Alkenylgruppe oder eine $C_3$-$C_6$-Alkinylgruppe;

$R^6$

Wasserstoff, eine $C_1$-$C_6$-Alkylgruppe, oder gemeinsam mit $R^5$ eine $C_4$-$C_6$-Alkylenkette, worin eine Methylengruppe durch ein Sauerstoffatom oder eine $C_1$-$C_4$-Alkyliminogruppe ersetzt sein kann, bedeutet.

Des weiteren betrifft die Erfindung ein Verfahren zur Herstellung der Verbindungen I sowie ihre Verwendung als herbizide Mittel und Zwischenprodukte zur Herstellung der Sulfonylharnstoffe I.

In den EP-A-84 020 und 169 815 werden Sulfonylharnstoffe beschrieben, die im Pyrimidinteil durch den Difluormethoxy- bzw. den Bromtrifluormethoxyrest substituiert sind. Diese Verbindungen lassen jedoch wegen der unbefriedigenden Selektivität gegen Schadpflanzen zu wünschen übrig. Entsprechend substituierte Triazine wurden bisher nicht bekannt.

Der Erfindung lagen daher neue Verbindungen aus der Klasse der Sulfonyl-1,3,5-triazin-2-yl-harnstoffe mit verbesserten herbiziden Eigenschaftn als Aufgabe zugrunde. Entsprechend dieser Aufgabe wurden die eingangs definierten Sulfonylharnstoffe gefunden.

Ferner wurde gefunden, daß die Verbindungen der Formel I sowie deren Alkali- und Erdalkalimetallsalze eine gute Selektivität gegen Schadpflanzen in Kulturen wie Getreide und Erdnüssen haben.

Außerdem wurden chemisch eigenartige Verfahren zur Herstellung der Verbindungen I gefunden. Im Vergleich zu dem Stand der Technik lassen sich die Sulfonylharnstoffe I in hoher Ausbeute und Reinheit herstellen, wenn man ausgeht von substituierten 2-Amino-4-fluoralkoxy-1,3,5-triazinen der allgemeinen Formel IIIa

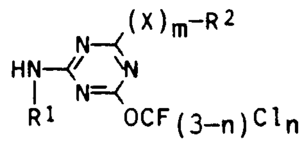

IIIa,

in der m für 1 und n für 0 oder 1 stehen und die Substituenten folgende Bedeutung haben:

$R^1$

Wasserstoff, $C_1$-$C_4$-Alkyl, $C_3$-$C_6$-Alkenyl oder $C_3$-$C_6$-Alkinyl ;

$R^2$

$C_1$-$C_4$-Alkyl, $C_3$-$C_6$-Alkenyl oder $C_3$-$C_6$-Alkinyl;

X

O, S oder N-$R^4$, wobei

$R^4$

für Wasserstoff oder $C_1$-$C_4$-Alkyl steht.

Diese Zwischenprodukte und deren Herstellung sind daher ebenfalls Gegenstand der Erfindung.

Zur Herstellung von im 1,3,5-Triazinteil halogensubstituierten Verbindungen ($R^2$ = Hal, m = 0) geht man von entsprechend substituierten 2-Amino-4-fluoralkoxy-6-halogen-1,3,5-triazinen der Struktur IIIb aus (s. Formelschema 2), deren Herstellung Gegenstand der zeitgleichen Anmeldung P 40 24 761 (O.Z. 0050/41799) ist. 1,3,5-Triazinzwischenprodukte mit m = 0 und $R^2$ = Trifluormethyl erhält man in analoger Weise gemäß Formelschema 3.

Die erfindungsgemäßen Sulfonylharnstoffe der Formel I sind auf den in Formelschema 1 beschriebenen Wegen A, B und C zugänglich:

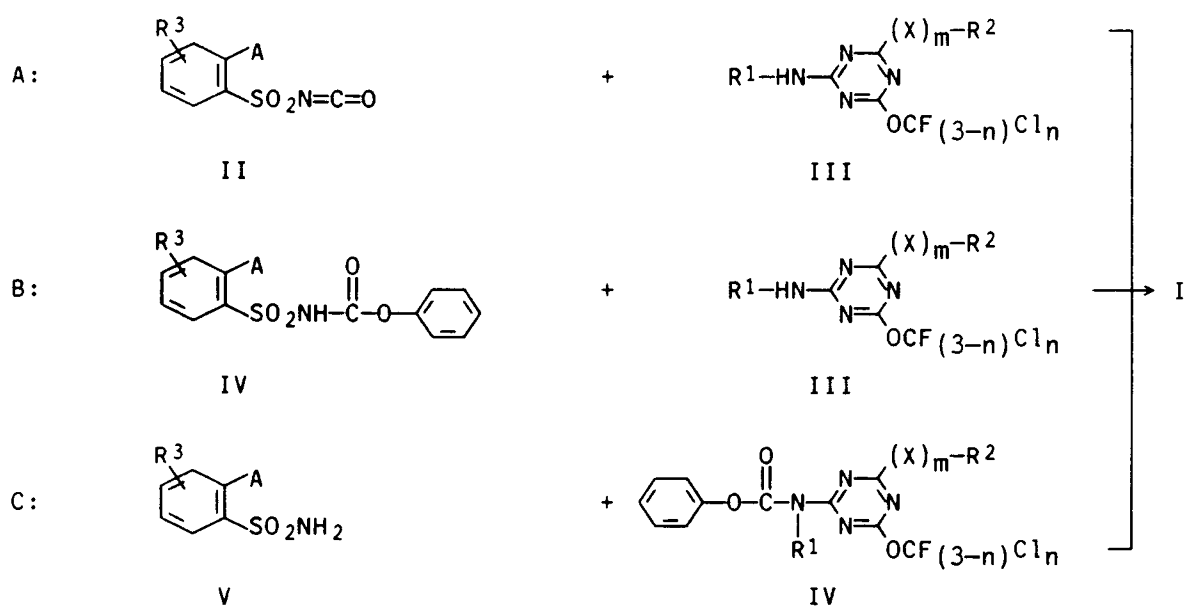

A: II + III

B: IV + III → I

C: V + IV

Ausführungsform A

Man setzt ein Sulfonylisocyanat II in an sich bekannter Weise (EP-A-162 723) in einem inerten organischen Lösungsmittel mit ungefähr der stöchiometrischen Menge eines 2-Amino-1,3,5-triazinderivats III bei einer Temperatur von 0 bis 120 °C, vorzugsweise 10 bis 100 °C um. Die Reaktion kann drucklos oder unter Druck (bis 50 bar), vorzugsweise bei 1 bis 5 bar, kontinuierlich oder diskontinuierlich durchgeführt werden. Geeignete Lösungsmittel sind in der obengenannten Literatur aufgeführt.

Ausführungsform B

Man setzt ein entsprechendes Sulfonylcarbamat der Formel IV in an sich bekannter Weise (EP-A-162 723) in einem inerten organischen Lösungsmittel bei einer Temperatur zwischen 0 und 120 ° C, vorzugsweise 10 bis 100 ° C mit einem 2-Amino-1,3,5-triazinderivats III um. Es können hierbei Basen wie tertiäre Amine zugesetzt werden, wodurch die Reaktion beschleunigt und die Produktqualität verbessert werden.

Geeignete Basen hierfür sind z. B. tertiäre Amine wie Pyridin, die Picoline, 2,4- und 2,6-Lutidin, 2,4,6-Collidin, p-Dimethylaminopyridin, 1,4-Diaza(2,2,2)bicyclooctan (DABCO) und 1,8-Diazabicyclo(5,4,0)-undec-7-en.

Zweckmäßig verwendet man als Lösungsmittel die in der Literatur angegebenen und/oder Halogenkohlenwasserstoffe wie Dichlormethan und Chlorbenzol, Ether wie Diethylether, Tetrahydrofuran und Dioxan, Acetonitril, Dimethylformamid und/oder Ester wie Essigsäureethylester in einer Menge von 100 bis 4000 Gew.%, vorzugsweise von 1000 bis 2000 Gew.%, bezogen auf die Ausgangsstoffe II, IV und V.

Im Rahmen der Herstellung der erfindungsgemäßen Verbindungen sind die 2-Amino-1,3,5-triazin-Zwischenprodukte III auf folgende vorteilhafte Weise zugänglich:

Formelschema 2:

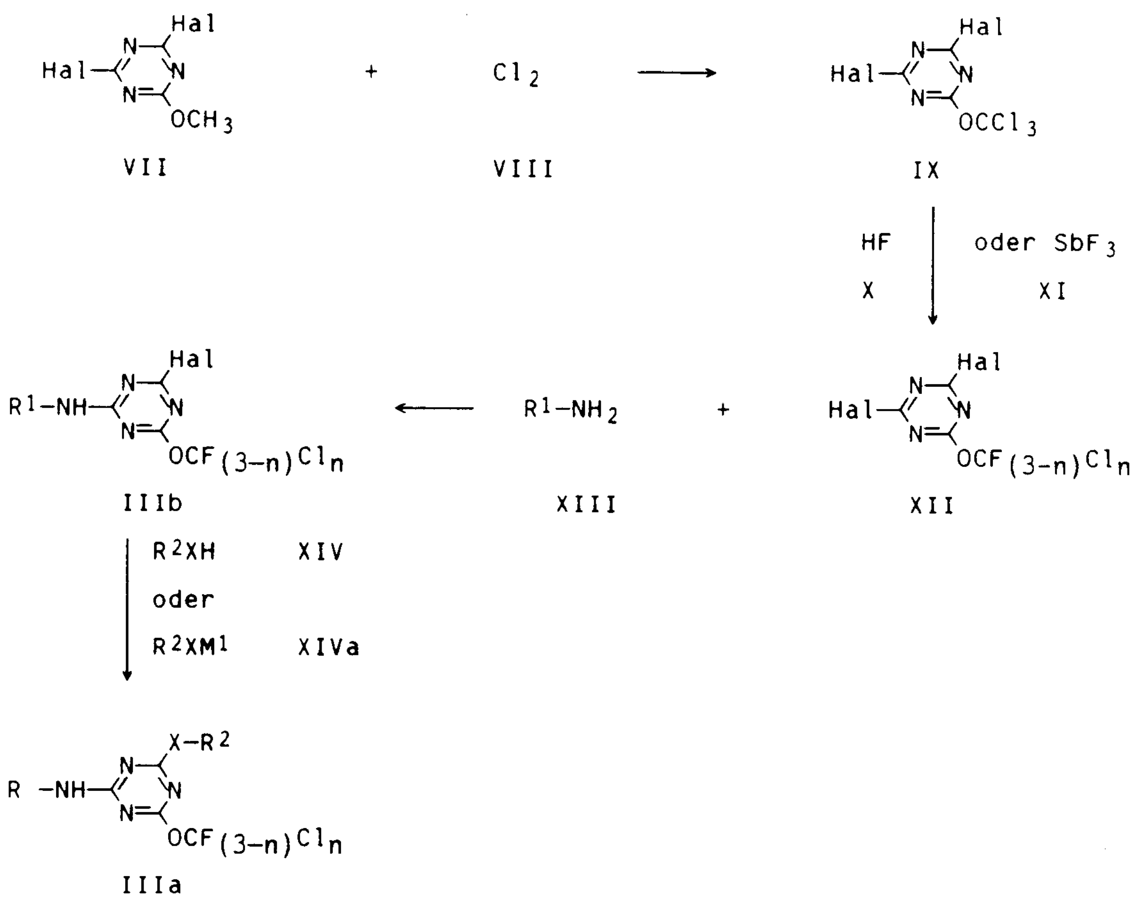

VII
VIII
IX
XII
XIII
IIIb
IIIa

In entsprechender Weise gelangt man zu den 2-Amino-6-trifluormethyl-1,3,5-triazinderivaten IIIc, wenn man 2,4-Dihalogen-6-trichlormethyl-1,3,5-triazine gemäß Formelschema 3 umsetzt.

Formelschema 3:

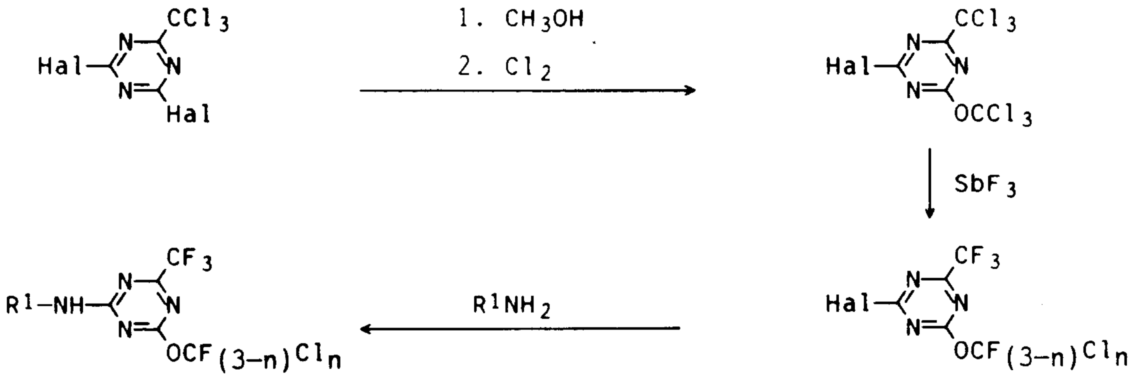


Ausgehend von den Zwischenprodukten XII in Formelschema 2 und Substitution des Halogenatoms in 4-Position durch die in Formelschema 3 beschriebene Reaktionssequenz (1. $CH_3OH$, 2. $Cl_2$, 3. $SbF_3$) sowie anschließende Umsetzung mit $R^1NH_2$ gelangt man zu den Zwischenprodukten IIId

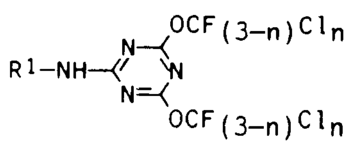
IIId

Die Chlorierung des 2-Methoxy-1,3,5-triazins VII mit Chlor VIII zu dem Trichlormethoxy-1,3,5-triazin IX führt man beispielsweise bei einer Temperatur von 100 bis 180 °C durch.

Als Chlorierungsmittel sind elementares Chlor oder Chlor abgebende Substanzen wie Sulfurylchlorid oder Phosphorpentachlorid geeignet. Chlor kann dabei auch in situ durch Oxydation von Chlorwasserstoffsäure, beispielsweise mit Wasserstoffsuperoxid hergestellt werden.

Die Umsetzung kann in Gegenwart eines inerten Lösungsmittels, beispielsweise einem chlorierten Kohlenwasserstoff wie Chlorbenzol, 1,2-, 1,3- oder 1,4-Dichlorbenzol, einer Nitroverbindung wie Nitrobenzol, einer Carbonsäure wie Essigsäure, Propionsäure, einem Säureanhydrid wie Essigsäureanhydrid, einem Säurechlorid wie Chloracetylchlorid, α-Chlorpropionsäurechlorid, α,α-Dichlorpropionsäurechlorid, einem anorganischen Säurehalogenid wie Phosphortrichlorid oder Phosphoroxychlorid oder bevorzugt ohne Lösungsmittel in der Schmelze des Ausgangsstoffes VII durchgeführt werden.

Gegebenenfalls kann man die Reaktion durch Zugabe eines Radikalstarters beschleunigen; als solcher eignet sich die Bestrahlung mit Licht, vorzugsweise UV-Licht oder die Zugabe von α,α'-Azoisobutyronitril in zweckmäßig einer Menge von 0,2 bis 7 Mol%, bezogen auf den Ausgangsstoff VII. Man kann die Reaktion auch durch Zugabe eines Katalysators beschleunigen; als solcher eignet sich Phosphorpentachlorid, zweckmäßig in einer Menge von 0,5 bis 7 Mol% bezogen auf den Ausgangsstoff VII. In diesem Fall legt man den Ausgangsstoff VII zusammen mit dem Katalysator vor und beginnt dann mit der Chlorierung. Statt des Phosphorpentachlorids kann man auch eine dieses unter den Reaktionsbedingungen bildende Ausgangskomponente, z.B. Phosphortrichlorid oder gelben Phosphor, zugeben und dann mit der Chlorierung beginnen.

Ausgangsstoff VII kann mit Chlor in annähernd stöchiometrischer Menge oder vorzugsweise im Überschuß, vorteilhaft mit 3,1 bis 11, insbesondere 3,3 bis 5 mol $Cl_2$ je Equivalent Methoxy in dem Ausgangsstoff VII umgesetzt werden. Die Umsetzung kann bei einer Temperatur von 100 bis 180 °C, vorteilhaft von 120 bis 150 °C, drucklos oder unter Druck kontinuierlich oder diskontinuierlich durchgefürt werden.

Chloriert man bei 1 bar, so werden zweckmäßig 3,3 bis 5 mol Chlorgas, bezogen auf ein Equivalent Methoxy in dem Ausgangsstoff VII, eingesetzt, was einem Chlorumsatz von 91 bis 60 % entspricht. Durch geeignete apparative Maßnahmen, z. B. durch Anwendung von mäßigem Überdruck, zweckmäßig 1 bis 10 bar, oder durch Verwendung einer Blasensäule, läßt sich der Chlorumsatz erhöhen. Vorteilhaft läßt man das Chlorgas möglichst lange mit der organischen Phase in Berührung kommen, indem diese beispielsweise stark gerührt wird oder das Chlorgas eine dicke Schicht der organischen Phase durchdringen muß.

Die Reaktionszeit beträgt im allgemeinen etwa 0,5 bis 12 Stunden.

In einer bevorzugten Ausführungsform des Verfahrens geht man so vor, daß man innerhalb von 0,5 bis 12 Stunden, vorzugsweise 1 bis 10 Stunden die erforderliche Menge Chlorgas unter intensivem Rühren in den flüssigen Ausgangsstoff VII einleitet, wobei man zunächst bei einer Temperatur von 120 bis 130 °C beginnt und diese - gegebenenfalls unter Ausnutzung des exothermen Charakters der Reaktion - kontinuierlich steigert, so daß gegen Ende die Umsetzung bei einer Temperatur von 135 bis 150 °C durchgeführt wird. Bei größeren Reaktionsansätzen muß dem exothermen Charakter durch äußere Kühlung bzw. geeignete Dosierung der Chlormenge Rechnung getragen werden; mit dem Abflauen der Reaktion entfernt man das Kältebad und kann gegebenenfalls noch nacherhitzen.

Die Aufarbeitung und Isolierung der Endstoffe kann in üblicher Weise erfolgen. Beispielsweise kann man aus der heißen organischen Phase mittels eines Inertgases Reste an Chlorwasserstoff, Chlor oder Katalysator austragen; dabei hinterbleibt in hoher Ausbeute ein bereits recht reines Rohprodukt. Durch Destillation oder Chromatographie kann es weiter gereinigt oder aber direkt für weitere Umsetzungen eingesetzt werden.

Die Umsetzung des Trichlormethoxy-1,3,5-triazins IX mit einem Halogenaustauschmittel führt man beispielsweise bei einer Temperatur von 0 bis 180 °C durch.

Als Halogenaustauschmittel sind Antimontrifluorid in Gegenwart oder Abwesenheit katalytischer Mengen eines Antimon(V)salzes oder Fluorwasserstoff geeignet.

Zweckmäßig verwendet man einen überschuß von 1 bis 200, vorzugsweise 5 bis 25 mol-% Antimontrifluorid pro Trichlormethylequivalent. Die Katalysatormenge an Antimon(V)salz beträgt zwischen 1 bis 20, vorzugsweise 5 bis 18 mol-% pro Trichlormethylequivalent. Vorzugsweise dosiert man den Ausgangsstoff XI bei 90 bis 130 °C zu der Mischung des Halogenaustauschmittels und erwärmt dann noch zwischen 10 und ca. 240 Minuten auf eine Temperatur zwischen 110 bis 180 °C. Anschließend wird durch Destillation aufgearbeitet.

Man kann die Reaktion jedoch auch kontinuierlich führen, den Ausgangsstoff XI bei 110 bis 180 °C innerhalb 10 bis ca. 240 Minuten zugeben und gleichzeitig unter vermindertem Druck den entstehenden niedriger siedenden Endstoff XIV abdestillieren. Spuren mitgerissener Antimonsalze lassen sich durch Extraktion mit konz. Salzsäure beseitigen.

Arbeitet man ohne Antimon(V)salz-Katalyse oder setzt nur geringe Mengen, z. B. 0,5 bis 5 mol-% ein, und reduziert die Menge an Antimontrifluorid auf 60 bis 90 mol-% pro Trichlormethylequivalent, so bleibt der Halogenaustausch auf der Chlordifluormethoxystufe stehen.

Anstelle von Antimontrifluorid kann der Halogenaustausch auch mit Fluorwasserstoff bei 0 bis 150 °C, vorzugsweise 40 bis 120 °C, durchgeführt werden. Hierzu versetzt man in einem Autoklaven den Ausgangsstoff IX mit einem überschuß von 300 bis 700, vorzugsweise 350 bis 400 mol-% Fluorwasserstoff pro Trichlormethylequivalent und rührt 10 Minuten bis 10 Stunden. Gegebenenfalls kann die Reaktion in gleicher Weise, wie für die Verwendung von Antimontrifluorid beschrieben, durch Zusatz eines Katalysators wie Antimonpentachlorid beschleunigt werden. Nach dem Entspannen und der Entfernung flüchtiger Bestandteile wird wie beschrieben aufgearbeitet.

Die Umsetzung des Fluormethoxy-1,3,5-triazins XII mit einem Amin XIII führt man beispielsweise bei einer Temperatur von -80 bis 40 °C durch.

In Formel XIII steht $R^1$ für Wasserstoff, $C_1$-$C_4$-Alkyl wie Methyl, Ethyl, n-Propyl, i-Propyl, n-Butyl, sek.-Butyl, i-Butyl, tert.-Butyl; $C_3$-$C_4$-Alkenyl wie 2-Propenyl, 2-Methylethenyl, 2-Butenyl, 3-Butenyl, 1-Methyl-2-propenyl oder 2-Methyl-2-propenyl ; $C_3$-$C_4$-Alkinyl wie Propargyl, 2-Butinyl, 3-Butinyl oder 1-Methyl-2-propinyl.

Unter den Aminen, welche eingesetzt werden können, sollen die folgenden erwähnt werden: Ammoniak, Methylamin, Ethylamin, n-Propylamin, Isopropylamin, n-Butylamin, Isobutylamin, sek.-Butylamin, tert.-Butylamin, 2-Propenylamin, 2-Methylethenylamin, 2-Butenylamin, 3-Butenylamin, 1-Methyl-2-propenylamin, 2-Methyl-2-propenylamin, Propargylamin, 2-Butinylamin, 3-Butinylamin und 1-Methyl-2-propinylamin.

Die 2-Halogen-1,3,5-triazine XII können in einem aprotisch polaren Lösungsmittel mit den Aminen XIII bei einer Temperatur von -80 bis 40 °C umgesetzt werden, wobei man das Amin XIII entweder in einem Überschuß einsetzt oder eine organische Hilfsbase verwendet.

Für die Umsetzung des 2,4-Dihalogen-1,3,5-triazins XII mit dem Amin XIII sind folgende Lösungsmittel geeignet:

Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäßig verwendet man das Lösungsmittel in einer Menge von 100 bis 2000 Gew.%, vorzugsweise 400 bis 1200 Gew.%, bezogen auf den Ausgangsstoff XII.

Vorteilhaft gibt man 1,8 bis 2,5, insbesondere 1,95 bis 2,2 mol-Equivalent des Amins XIII, bezogen auf den Ausgangsstoff XII innerhalb 0,5 bis 2 Stunden zu einer Mischung von Ausgangsstoff XII in einem der vorgenannten Lösungsmittel bei (-80 °C) bis 40 °C, vorzugsweise -70 bis 25 °C, rührt bis zur Vervollständigung der Reaktion bis zu 3 Stunden nach und läßt dann zur Aufarbeitung auf 25 °C erwärmen.

Setzt man nur ungefähr stöchiometrische Mengen des Amins XIII ein, so verwendet man zweckmäßig 0,9 bis 1,1, Equivalente einer organischen Hilfsbase, bezogen auf den Ausgangsstoff XII. Als Hilfsbase eignen sich organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, $\alpha$-,$\beta$-,$\gamma$-Picolin, 2,4- und 2,6-Lutidin und Triethylendiamin.

Die Reaktion kann drucklos oder unter Druck, kontinuierlich oder diskontinuierlich durchgeführt werden.

Zur Aufarbeitung extrahiert man das Umsetzungsgemisch mit Wasser zur Entfernung der Salze, trocknet und reinigt die organische Phase, z. B. durch Chromatographie. Man kann jedoch auch direkt die organische Phase einengen und den Rückstand mit einem Lösungsmittel verrühren.

Die erfindungsgemäßen 2-Amino-4-fluoralkoxy-1,3,5-triazine der Formel IIIa erhält man vorteilhaft in der Weise, daß man 2-Amino-4-fluoralkoxy-6-halogen-1,3,5-triazine der Formel IIIb

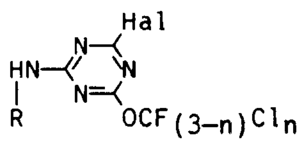

IIIb

in der Hal für Fluor, Chlor oder Brom steht und $R^1$ und n die vorgenannte Bedeutung haben, mit einem Nucleophil der Formel XIV

$H\text{-}X\text{-}R^2$ XIV

in der X und $R^2$ die vorgenannte Bedeutung besitzen, oder dessen Salz umsetzt.

Die Umsetzung kann für den Fall der Verwendung von 2-Amino-4-fluor-6-trifluormethoxy-1,3,5-triazin und Methylamin durch folgendes Schema beschrieben werden:

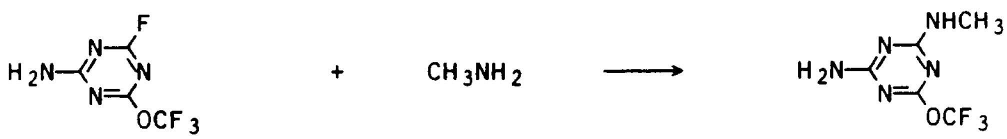

Für den Fall der Verwendung von 2-Amino-4-fluor-6-chlordifluormethoxy-1,3,5-triazin und Natriummethylat läßt sich die Umsetzung durch folgendes Schema wiedergeben:

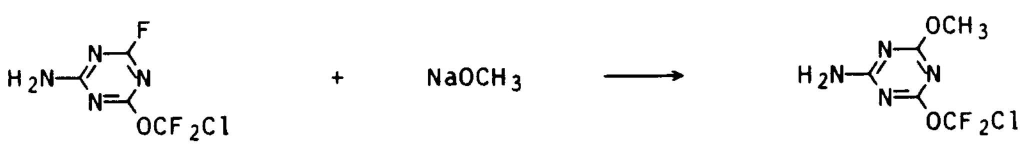

Das Verfahren liefert auf einfachem und wirtschaftlichem Weg neue 2-Amino-4-fluoralkoxy-1,3,5-triazine in hoher Ausbeute und Reinheit. Entgegen der Erwartung werden Fluoralkoxygruppen nicht substituiert. Auch das in der Etherseitenkette stehende Chloratom bleibt trotz der alkalischen Reaktionsbedingungen erhalten. Im Hinblick auf den Stand der Technik (s. z.B. EP-A-70 804) sind alle diese vorteilhaften Eigenschaften überraschend.

Bevorzugte Zwischenprodukte IIIa und dementsprechend bevorzugte Ausgangsstoffe IIIb sind solche, in deren Formeln die Substituenten folgende Bedeutung haben:

$R^1$ und $R^2$ $C_1$-$C_4$-Alkyl wie Methyl, Ethyl, n-Propyl, i-Propyl, n-Butyl, sek.-Butyl, i-Butyl, tert.-Butyl; $C_3$-$C_4$-Alkenyl wie 2-Propenyl, 2-Methyl-ethenyl, 2-Butenyl, 3-Butenyl, 1-Methyl-2-propenyl, 2-Methyl-2-propenyl; $C_3$-$C_4$-Alkinyl wie Propargyl, 2-Butinyl, 3-Butinyl, 1-Methyl-2-propinyl, darüber hinaus kann $R^1$ auch

Wasserstoff bedeuten und
X
O, S oder N-$R^4$, wobei
$R^4$
für Wasserstoff, Methyl, Ethyl, n-Propyl, i-Propyl, n-Butyl, sek.-Butyl, i-Butyl oder tert.-Butyl steht und
n
0 oder 1
darstellt.

Die Umsetzung des 2-Amino-4-fluoralkoxy-1,3,5-triazins IIIb mit einem Nucleophil XIV oder dessen Salz XIVa führt man beispielsweise bei einer Temperatur von -80 bis 80 °C durch. Als Nucleophile XIV sind Ammoniak, aliphatische Amine, Alkohole und Thiole geeignet.

Unter den Aminen, welche als Nucleophile eingesetzt werden können, sollen die folgenden erwähnt werden: Ammoniak, Methylamin, Ethylamin, n-Propylamin, Isopropylamin, n-Butylamin, Isobutylamin, sek.-Butylamin, tert.-Butylamin, 2-Propenylamin, 2-Methylethenylamin, 2-Butenylamin, 3-Butenylamin, 1-Methyl-2-propenylamin, 2-Methyl-2-propenylamin, Propargylamin, 2-Butinylamin, 3-Butinylamin und 1-Methyl-2-propinylamin, Dimethylamin, Diethylamin, Di-n-propylamin, Di-n-butylamin, N-Methyl-ethylamin, N-Ethyl-n-propylamin, N-Methyl-allylamin und N-Methyl-propargylamin.

Unter den Alkoholen, welche als Nucleophile eingesetzt werden können, sollen die folgenden erwähnt werden: Methanol, Ethanol, n-Propanol, i-Propanol, n-Butanol, i-Butanol, sek.-Butanol, tert.-Butanol, 2-Propenol, 2-Methylethenol, 2-Butenol, 3-Butenol, 1-Methyl-2-propenol, 2-Methyl-2-propenol, Propinol, 2-Butinol, 3-Butinol und 1-Methyl-2-propinol.

Unter den Thiolen, welche als Nucleophile eingesetzt werden können, sollen die folgenden erwähnt werden: Methanthiol, Ethanthiol, n-Propanthiol, i-Propanthiol, n-Butanthiol, i-Butanthiol, sek.-Butanthiol, tert.-Butanthiol, 2-Butenthiol, 2-Methylethenthiol, 2-Butenthiol, 3-Butenthiol, 1-Methyl-2-propenthiol, 2-Methyl-2-propenthiol, Propinthiol, 2-Butinthiol, 3-Butinthiol und 1-Methyl-2-propinthiol.

Die 4-Halogen-1,3,5-triazine IIIb können in einem aprotisch polaren Lösungsmittel mit den Aminen XIV bei einer Temperatur von -80 bis +80 °C, vorteilhaft -30 bis +20 °C umgesetzt werden, wobei man das Amin XIV entweder in einem überschuß einsetzt oder eine organische Hilfsbase verwendet.

Für die Umsetzung des 4-Halogen-1,3,5-triazins IIIb mit dem Amin XIV sind folgende Lösungsmittel geeignet:
Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäpig verwendet man das Lösungsmittel in einer Menge von 100 bis 2000 Gew.%, vorzugsweise 400 bis 1200 Gew.%, bezogen auf den Ausgangsstoff IIIb.

Vorteilhaft gibt man 1,8 bis 2,5, insbesondere 1,95 bis 2,2 mol-Äquivalente des Amins XIV, bezogen auf den Ausgangsstoff IIIb innerhalb 0,5 bis 2 Stunden zu einer Mischung von Ausgangsstoff IIIb in einem der vorgenannten Lösungsmittel bei (-80) bis 80 °C, vorzugsweise -30 bis 25 °C, rührt bis zur Vervollständigung der Reaktion (bis zu 3 Stunden) nach und läßt dann zur Aufarbeitung auf 25 °C erwärmen.

Setzt man nur ungefähr stöchiometrische Mengen des Amins XIV ein, so muß man zweckmäßig 0,9 bis 1,1 Equivalente einer organischen Hilfsbase, bezogen auf den Ausgangsstoff IIIb, zusetzen. Als Hilfsbase eignen sich organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, $\alpha$-,$\beta$-,$\gamma$-Picolin, 2,4- und 2,6-Lutidin und Triethylendiamin.

Wird die Umsetzung mit Alkoholen oder Thiolen durchgeführt, kann man analog der für Amine beschriebenen Reaktionsweise verfahren. Vorteilhaft gibt man das Nucleophil in einer Menge von 0,9 bis 1,3 mol-Äquivalenten bezogen auf den Ausgangsstoff IIIb innerhalb 0,5 bis 2 Stunden zusammen mit einer der vorgenannten Hilfsbasen zu einer Mischung von Ausgangsstoff IIIb in einem der vorgenannten Lösungsmittel bei -30 bis 20 °C, rührt bis zur Vervollständigung der Reaktion (bis zu 3 Stunden) nach und läßt dann zur Aufarbeitung auf 25 °C erwärmen.

Neben den genannten sind als Lösungsmittel auch Ketone; z. B. Aceton, Methylethylketon; dipolare aprotische Lösungsmittel, z. B. Acetonitril, Dimethylformamid, Dimethylacetamid, Dimethylsulfoxid, N-Methylpyrrolidon, 1,3-Dimethylimidazolin-2-on; Aromaten, z. B. Benzol, Toluol, Xylol oder entsprechende Gemische geeignet. Vorteilhaft kann man im Fall des Einsatzes von Alkoholen als Nucleophile diese direkt als Lösungsmittel verwenden. Besonders bevorzugt sind Salze von Alkoholen oder Thiolen, die den Einsatz einer organischen Hilfsbase entbehrlich machen. Sie werden auf bekannte Weise unter Verwendung von

Alkali- oder Erdalkalimetallen oder Metallhydriden, z. B. NaH, KH, $CaH_2$ oder LiH hergestellt.

Die Reaktion kann drucklos oder unter Druck, kontinuierlich oder diskontinuierlich durchgeführt werden.

Zur Aufarbeitung extrahiert man das Umsetzungsgemisch mit Wasser zur Entfernung der Salze, trocknet und reinigt die organische Phase, z. B. durch Chromatographie. Die Umsetzungsprodukte sind jedoch meist genügend rein, so daß man nur von dem ausgefallenen Salz abzufiltrieren und die organische Phase einzuengen braucht.

Bevorzugte Zwischenprodukte der Formel IIIa sind beispielsweise:

2-Amino-4-methoxy-6-trifluormethoxy-1,3,5-triazin
2-Amino-4-chlordifluormethoxy-6-methoxy-1,3,5-triazin
2-Amino-4-ethoxy-6-trifluormethoxy-1,3,5-triazin
2-Amino-4-chlordifluormethoxy-6-ethoxy-1,3,5-triazin
2-Amino-4-allyloxy-6-trifluormethoxy-1,3,5-triazin
2-Amino-4-allyloxy-6-chlordifluormethoxy-1,3,5-triazin
2-Amino-4-methylthio-6-trifluormethoxy-1,3,5-triazin
2-Amino-4-chlordifluormethoxy-6-methylthio-1,3,5-triazin
2-Amino-4-ethylthio-6-trifluormethoxy-1,3,5-triazin
2-Amino-4-chlordifluormethoxy-6-ethylthio-1,3,5-triazin
2-Amino-4-methylamino-6-trifluormethoxy-1,3,5-triazin
2-Amino-4-chlordifluormethoxy-6-methylamino-1,3,5-triazin
2-Amino-4-ethylamino-6-trifluormethoxy-1,3,5-triazin
2-Amino-4-chlordifluormethoxy-6-ethylamino-1,3,5-triazin
2-Amino-4-dimethylamino-6-trifluormethoxy-1,3,5-triazin
2-Amino-4-chlordifluormethoxy-6-dimethylamino-1,3,5-triazin
4-Methoxy-2-methylamino-6-trifluormethoxy-1,3,5-triazin
4-Chlordifluormethoxy-6-methoxy-2-methylamino-1,3,5-triazin
4-Ethoxy-2-methylamino-6-trifluormethoxy-1,3,5-triazin
4-Chlordifluormethoxy-6-ethoxy-2-methylamino-1,3,5-triazin
2,4-Bis-methylamino-6-trifluormethoxy-1,3,5-triazin
4-Chlordifluormethoxy-2,6-bis-methylamino-1,3,5-triazin
4-Ethylamino-2-methylamino-6-trifluormethoxy-1,3,5-triazin
4-Chlordifluormethoxy-6-ethylamino-2-methylamino-1,3,5-triazin
4-Dimethylamino-2-methylamino-6-trifluormethoxy-1,3,5-triazin
4-Chlordifluormethoxy-6-dimethylamino-2-methylamino-1,3,5-triazin

Ausführungsform C

Man setzt ein Sulfonamid der Formel V in an sich bekannter Weise (EP-A-141 777) in einem inerten organischen Lösungsmittel mit ungefähr der stöchiometrischen Menge eines Phenylcarbamats VI bei einer Temperatur von 0 bis 120 °C, vorzugsweise 20 bis 100 °C um. Die Reaktion kann drucklos oder unter Druck (bis 50 bar), vorzugsweise bei 1 bis 5 bar, kontinuierlich oder diskontinuierlich durchgeführt werden.

Geeignete Lösungsmittel sind neben den in der oben zitierten Literatur aufgeführten, z. B. Nitrokohlenwasserstoffe wie Nitroethan und Nitrobenzol, Nitrile wie Acetonitril und Benzonitril, Ester wie Essigsäureethylester, Amide wie Dimethylformamid und/oder Ketone wie Aceton. Bevorzugt wird die Umsetzung in Essigsäureethylester als Lösungsmittel und mit Pyridin oder einem der vorstehend genannten tertiären Amin als Base.

Die als Ausgangsstoffe der Formel V benötigten Sulfonamide lassen sich aus substituierten Anthranilestern durch Meerwein-Reaktion und anschließende Umsetzung mit Ammoniak herstellen.

Verbindungen der Formel I, in der $R^5$ Wasserstoff bedeutet, erhält man durch Hydrolyse von Estern der Formel I, in der $R^5$ einen $C_1$-$C_6$-Alkylrest bedeutet. Die Hydrolyse wird mit mindestens der doppelten Menge einer Base wie Natrium- oder Kaliumhydroxid, zweckmäßig in einem Lösungsmittelgemisch mit der 2- bis 8-fachen Menge Methanol und der 10- bis 40-fachen Menge Wasser, bezogen auf das Gewicht des entsprechenden Esters der Formel I, bei 30 bis 80 °C während 1 bis 20 Stunden durchgeführt. Durch Ansäuern fällt man die Sulfonamidcarbonsäuren der Formel I aus.

Im Hinblick auf die biologische Wirksamkeit sind Verbindungen der Formel I bevorzugt, in denen die Substituenten folgende Bedeutung haben:

$R^1$

Wasserstoff und Methyl;

$R^2$

Fluor, Chlor und Brom und Trifluormethyl, (m = 0), ferner Methyl, Ethyl, n-Propyl und Isopropyl (m = 1);
$R^3$
Wasserstoff, Fluor, Chlor, Brom, Methyl, Methoxy und Trifluormethyl;
X
Sauerstoff, Schwefel und eine Aminogruppe $-NR^4$, wobei
$R^4$
für Wasserstoff, Methyl und Ethyl steht;
A
Chlor, Trifluormethyl, CN, $NO_2$, Methyl, Ethyl, Methoxy, Ethoxy, Methylthio, Ethylthio, Methylsulfinyl, Methylsulfonyl, Ethylsulfonyl, eine Carboxylatgruppe und eine Carboxamidgruppe;
$R^5$
eine $C_1$-$C_6$-Alkylgruppe, insbesondere $C_1$-$C_4$-Alkylgruppe wie Methyl, Ethyl, n-Propyl und Isopropyl,
eine $C_2$-$C_4$-Alkenylgruppe wie Allyl, Crotyl und Buten-1-en-3-yl;
eine $C_2$-$C_4$-Alkinylgruppe wie Propargyl, But-1-in-3-yl und But-2-inyl;
Halogenalkyl wie 2-Chlorethyl, 2-Chlor-n-propyl, 3-Chlor-n-propyl, 1-Chlorbut-2-yl, 2-Chlor-iso-butyl, 4-Chlor-n-butyl, Chlor-tert.-butyl, 3-Chlor-prop-2-yl und 2,2,2-Trifluorethyl;
Alkoxyalkyl wie 2-Methoxyethyl, 3-Ethoxyethyl, 3-Methoxy-n-propyl, 2-Methoxy-n-propyl, 3-Methoxy-n-butyl, 1-Methoxy-but-2-yl, Methoxy-tert.-butyl, 2-Methoxy-n-butyl und 4-Methoxy-n-butyl;
Alkoxyalkoxyalkyl wie 2-Methoxy-ethoxy-methyl, 2-(Ethoxy)-ethoxy-methyl, 2-(Propoxy)-ethoxy-methyl, 2-Methoxy-ethoxy-ethyl, 2-(Ethoxy)-ethoxy-ethyl und 2-(Methoxy-methoxy)-ethyl;
Halogenalkoxyalkyl wie 2-($\beta$-Chlorethoxy)-ethyl, 3-($\beta$-Chlorethoxy)-n-propyl und 3-($\gamma$-Chlor-n-propoxy)-n-propyl;
Cycloalkyl wie Cyclopentyl und Cyclohexyl,
$R^6$
Wasserstoff;
$C_1$-$C_6$-, insbesondere $C_1$-$C_4$-Alkyl wie Methyl, Ethyl, n-Propyl, Isopropyl und n-Butyl oder gemeinsam mit $R^5$ Tetramethylen, Pentamethylen, Hexamethylen, Ethylenoxyethylen und Ethlyen-N-methylimino-ethylen und
n
0 oder 1.

Als Salze der Verbindungen der Formel I kommen landwirtschaftlich brauchbare Salze, beispielsweise Alkalimetallsalze wie das Kalium-oder Natriumsalz, Erdalkalimetallsalze, wie das Calcium-, Magnesium-oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden und wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder öldispersionen. Als inerte Zusatzstoffe kommen Mineralölfraktionen von mittlerem bis hohem Siedepunkt, wie Kerosin oder Dieselöl, ferner Kohlenteeröle sowie öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder öldispersionen können die Substrate als solche oder in einem öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl-und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 % vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 5.019 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 5.019 werden in einer Mischung gelöst, die aus 80 Gewichtsteilen Xylol, 10 Gewichtsteilen des Anlagerungsproduktes von 8 bis 10 mol Ethylenoxid an 1 mol ölsäure-N-monoethanolamid, 5 Gewichtsteilen Calciumsalz der Dodecylbenzolsulfonsäure und 5 Gewichtsteilen des Anlagerungsproduktes von 40 mol Ethylenoxid an 1 mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 5.019 werden in einer Mischung gelöst, die aus 40 Gewichtsteilen Cyclohexanon, 30 Gewichtsteilen Isobutanol, 20 Gewichtsteilen des Anlagerungsproduktes von 7 mol Ethylenoxid an 1 mol Isooctylphenol und 10 Gewichtsteilen des Anlagerungsproduktes von 40 mol Ethylenoxid an 1 mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 5.019 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 mol Ethylenoxid an 1 mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 5.019 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 5.019 werden mit 97 Gewichtsteilen feinteiligem Kaolin vermischt. Man erhält auf diese Weise ein Stäubemittel, das 3 Gew.% des wirkstoffs enthält.

VII. 30 Gewichtsteile des Wirkstoffs Nr. 5.019 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 5.019 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 2, vorzugsweise 0,01 bis 1 kg/ha aktive Substanz (a.S.).

Die Verbindungen der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außer dem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen

Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

In Anbetracht der Vielseitigkeit der Applikationsmethoden können die erfindungsgemaßen Verbindungen bzw. sie enthaltende Mittel in einer großen Zahl von Kulturpflanzen zur Beseitigung unerwünschter Pflanzen eingesetzt werden.

Kulturenliste:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffel |
| Juglans regia | Walnußbaum |
| Lens culinaris | Linse |

| Botanischer Name | Deutscher Name |
|---|---|
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Micotiana tabacum (N. rustica) | Takak |
| Olea europaea | Ölbaum |
| Oryza sativa | Reis |
| Phaseolus lunatus | Mondbohne |
| Phaseolus vulgaris | Buschbohne |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazin, 4H-3,1-Benzoxazinderivate, Benzothiadiazinone, 2,6-Dinitroaniline, N-Phenylcarbamate, Thiocarbamate, Halogencarbonsäuren, Triazine, Amide, Harnstoffe, Diphenylether, Triazinone, Uracile, Benzofüranderivate, Cyclohexan-1,3-dionderivate, Chinolincarbonsäurederivate, Sulfonylharnstoffderivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische öle und ölkonzentrate zugesetzt werden.

Synthesebeispiele

I Herstellung der Vorprodukte

Beispiel I.1

2,4-Difluor-6-trichlormethoxy-1,3,5-triazin

In eine Mischung von 300 g (2,041 mol) 2,4-Difluor-6-methoxy-1,3,5-triazin und 0,3 g α,α'-Azoisobutyronitril leitete man bei 130 ° C und unter UV-Bestrahlung einen Strom von Chlorgas so ein, daß sich während 2 Stunden eine Temperatur von 140 bis 145 ° C einstellte. Nach NMR-spektroskopischer Kontrolle des Reaktionsverlaufs wurde unter äußerer Beheizung noch weitere 3 Stunden bei 135 bis 140 ° C mit Chlor begast.

Nach dem Absaugen von ausgefallenem Niederschlag und Destillation des Filtrats im Vakuum erhielt man 444 g (87 % d. Th.) der Titelverbindung vom Sdp. 40 bis 46 ° C/0,3 mbar.

Beispiel I.2

2,4-Difluor-6-trifluormethoxy-1,3,5-triazin

Zu einer Mischung von 187,4 g (1,048 mol) Antimontrifluorid und 35,2 g (0,117 mol) Antimonpentachlorid wurden die Hälfte von 210 g (0,838 mol) 2,4-Difluor-6-trichlormethoxy-1,3,5-triazin zunächst bei 110 ° C unter Rühren so zugegeben, daß sich anfangs eine Temperatur von 125 ° C einstellt; mit dem sich einstellenden Rückfluß mußte bei weiterer Zugabe außen beheizt werden. Es wurde eine Stunde bei 125 bis 130 ° C gerührt und eine bei 100 bis 105 ° C siedende Fraktion über eine 25-cm-Füllkörperkolonne abdestilliert. Nach dem Abklingen der Reaktion wurde die restliche Hälfte der Trichlormethoxyverbindung innerhalb 30 Minuten zugetropft und die bei 100 bis 105 ° C übergehende Fraktion kontinuerliche abdestilliert. Die Gesamtreaktionszeit betrug 3 Stunden. Man erhielt 134,4 g (79,8 % d. Th.) der Titelverbindung mit $n_D^{24}$ = 1.3650.

Beispiel I.3

6-Chlordifluormethoxy-2,4-difluor-1,3,5-triazin

210 g (0,838 mol) 2,4-Difluor-6-trichlormethoxy-1,3,5-triazin wurden innerhalb 10 Minuten unter Rühren bei 110 ° C zu 110 g (0,614 mol) Antimontrifluorid gegeben. Nach Zugabe von 3/4 von 9,38 g (0,0313 mol) Antimonpentachlorid wurde auf 145 ° C erwärmt und 1 Stunde gerührt. Restlicher Katalysator wurde zugegeben und nochmals 2 Stunden gerührt, wobei als niedersiedende Fraktion über eine 30-cm-Füllkörperkolonne zwischen 95 bis 105 ° C 20 g (11,8 % d. Th.) 2,4-Difluor-6-trifluormethoxy-1,3,5-triazin erhalten wurde. Der Destillationsrückstand wurde ohne Kolonne destilliert und ergab 94,8 g (52 % d. Th.) der Titelverbindung vom Sdp. 125 bis 130 ° C; $n_D^{24}$ = 1.4042.

Beispiel I.4

2,4-Dichlor-6-trifluormethoxy-1,3,5-triazin

Zu einer Mischung von 40,9 g (0,229 mol) Antimontrifluorid und 7,03 g (0,0234 mol) Antimonpentachlorid wurden 52 g (0,183 mol) 2,4-Dichlor-6-trichlormethoxy-1,3,5-triazin innerhalb 5 Minuten unter Rühren bei 90 ° C zugegeben, wobei sich die Temperatur bis auf 180 ° C erhöhte. Es wurde noch 20 Minuten bei 170 bis 180 ° C nachgerührt und dann das Rohprodukt bei 90 bis 103 ° C/70 mbar abdestilliert. Durch nochmalige Destillation erhielt man 32,3 g (75,5 % d. Th.) der Titelverbindung vom Sdp. 165 bis 173 ° C.

Beispiel I.5

2-Amino-4-fluor-6-trifluormethoxy-1,3,5-triazin

4,4 g (0,259 mol) Ammoniakgas wurden innerhalb 45 Minuten bei -70 bis -65 ° C unter Rühren in eine Mischung von 26,0 g (0,1293 mol) 2,4-Difluor-6-trifluormethoxy-1,3,5-triazin in 100 ml Tetrahydrofuran

eingeleitet. Es wurde 2 Stunden bei -70 °C und über Nacht unter Erwärmung bis auf 22 °C gerührt. Nach dem Einengen im Vakuum wurde der Rückstand mit Wasser verrührt, abgesaugt und gewaschen. Nach dem Trocknen erhielt man 22 g (85,9 % d. Th.) der Titelverbindung vom Fp. 138 bis 139 °C.

Beispiel I.6

2,4-Bismethylamino-6-trifluormethoxy-1,3,5-triazin und 2-Methylamino-4-fluor-6-trifluormethoxy-1,3,5-triazin

5,9 g (0,189 Mol) Methylamin wurden bei -70 °C innerhalb 30 Minuten unter Rühren in eine Mischung von 19,0 g (0,0945 Mol) 2,4-Difluor-6-trifluormethoxy-1,3,5-triazin in 100 ml Diethylether eingegast. Es wurde 2 Stunden bei -70 °C und über Nacht unter Erwärmung bis auf 22 °C gerührt. Das Reaktionsgemisch wurde im Vakuum eingeengt, in Methylenchlorid aufgenommen und mit Wasser gewaschen. Nach dem Trocknen wurde über eine Kieselgelsäule fraktioniert chromatographiert, wobei man in den ersten beiden Fraktionen 5,0 g (25 % d. Th.) 2-Methylamino-4-fluor-6-trifluormethoxy-1,3,5-triazin vom Fp. 68 bis 72 °C erhielt. In den weiteren Fraktionen 4 bis 7 isolierte man 10,7 g (51 % d. Th.) des schwerer löslichen 2,4-Bismethylamino-6-trifluormethoxy-1,3,5-triazins vom Fp. 150 bis 152 °C.

Beispiel I.7

2-Amino-4-chlordifluormethoxy-6-fluor-1,3,5-triazin und 2,4-Diamino-6-chlordifluormethoxy-1,3,5-triazin

7,8 g (0,46 Mol) Ammoniak wurden innerhalb 45 Minuten unter Rühren bei -70 °C in eine Mischung von 50,0 g (0,23 Mol) 2,4-Difluor-6-chlordifluormethoxy-1,3,5-triazin in 150 ml Tetrahydrofuran eingeleitet. Es wurde 2 Stunden bei -70 °C und über Nacht unter Erwärmung bis auf 22 °C gerührt. Das Reaktionsgemisch wurde im Vakuum eingeengt, mit Wasser gewaschen und getrocknet. Anschließend wurde das Reaktionsprodukt mit Methylenchlorid auf eine Kieselgelsäule geschlämmt und mit gleichem Lösungsmittel eluiert. In den Fraktionen 1 bis 8 erhielt man 21,5 g (43,6 % d. Th.) 2-Amino-4-fluor-6-chlordifluormethoxy-1,3,5-triazin vom Fp. 131 -133 °C.

Durch Nachspülen mit Essigester isolierte man dann in den Fraktionen 9 bis 14 das schwerer lösliche 2,4-Diamino-6-chlordifluormethoxy-1,3,5-triazin (11,2 g, 23 % d. Th.) vom Fp. 114 °C.

Beispiel I.8

2-Chlordifluormethoxy-4-fluor-6-methylamino-1,3,5-triazin und 2,4-Bis-methylamino-6-chlordifluormethoxy-1,3,5-triazin

5,2 g (0,166 mol) Methylamin wurden innerhalb 20 Minuten unter Rühren bei -70 °C in eine Mischung von 18,1 g (0,083 mol) 4-Difluorchlormethoxy-2,6-difluor-1,3,5-triazin und Lösungsmittel eingeleitet. Es wurde 2 Stunden bei -70 °C und über Nacht unter Erwärmung bis auf 22 °C gerührt. Das Reaktionsgemisch wurde im Vakuum eingeengt, in Methylenchlorid aufgenommen, mit Wasser gewaschen und getrocknet. Nach Chromatographie über Kieselgel erhielt man in den ersten Fraktionen 5,5 g (29 % d. Th.) 2-Chlordifluormethoxy-4-fluor-6-methylamino-1,3,5-triazin vom Fp. 62 -64 °C. Im Nachlauf isolierte man 8,7 g (44 % d. Th.) 2,4-Bismethylamino-6-chlordifluormethoxy-1,3,5-triazin vom Fp. 118 bis 120 °C.

II Herstellung der Zwischenprodukte IIIa

Beispiel II.1

2-Amino-4-methoxy-6-trifluormethoxy-1,3,5-triazin

9,1 g (0,05 mol) 30%iges Natriummethylat wurden bei 0 °C innerhalb 15 Minuten unter Rühren zu einer Mischung von 10 g (0,05 mol) 2-Amino-4-fluor-6-trifluormethoxy-1,3,5-triazin in 100 ml Methanol gegeben. Nach einer Stunde Rühren bei 0 °C wurde im Vakuum eingeengt, in Methylenchlorid aufgenommen und mit Wasser extrahiert. Nach dem Trocknen und Einengen erhielt man 10,5 g (99 % d. Th.) der Titelverbindung vom Fp. 96 bis 101 °C.

Beispiel II.2

2-Amino-4-chlordifluormethoxy-6-methoxy-1,3,5-triazin

8,4 g (0,047 mol) 30%iges Natriummethylat wurden bei 0 °C innerhalb 15 Minuten unter Rühren zu einer Mischung von 10 g (0,047 mol) 2-Amino-4-chlordifluormethoxy-6-fluor-1,3,5-triazin in 100 ml Methanol gegeben. Nach einer Stunde Rühren bei 0 °C wurde im Vakuum eingeengt, in Methylenchlorid aufgenommen und mit Wasser extrahiert. Nach dem Trocknen und Einengen erhielt man 10,4 g (98,5 % d. Th.) der Titelverbindung vom Fp. 109 bis 111 °C.

Beispiel II.3

2-Amino-4-ethoxy-6-trifluormethoxy-1,3,5-triazin

2,3 g (0,093 Mol) 97 % Natriumhydrid wurden portionsweise bei 20 bis 35 °C zu 300 ml Ethanol gegeben und 15 Minuten bis zur Lösung gerührt. Bei 0 °C wurden dann unter Rühren 18,5 g (0,093 Mol) 2-Amino-4-fluor-6-trifluormethoxy-1,3,5-triazin innerhalb 10 Minuten zugegeben, 1 Stunde bei 0 °C und über Nacht bei 22 °C gerührt. Nach dem Einengen im Vakuum wurde der Rückstand in Methylenchlorid aufgenommen, mit Wasser extrahiert und getrocknet. Nach dem Einengen erhielt man 17,9 g (85,9 % d. Th.) der Titelverbindung vom Fp. 69 bis 71 °C.

Beispiel II.4

2-Amino-4-chlordifluormethoxy-6-ethoxy-1,3,5-triazin

1,2 g (0,047 mol) 97 % Natriumhydrid wurden bei 20 bis 35 °C portionsweise zu 150 ml Ethanol gegeben und 15 Minuten bis zur Lösung gerührt. Anschließend wurde bei 0 °C unter Rühren 10,0 g (0,047 mol) 2-Amino-4-chlordifluormethoxy-6-fluor-1,3,5-triazin zugegeben, 1 Stunde bei 0 °C und über Nacht bei 22 °C gerührt. Nach dem Einengen im Vakuum wurde der Rückstand in Methylenchlorid aufgenommen, mit Wasser extrahiert und getrocknet. Nach dem Einengen erhielt man 10,6 g (94,6 % d. Th.) der Titelverbindung vom Fp. 63 bis 65 °C.

Beispiel II.5

2-Amino-4-methylamino-6-trifluormethoxy-1,3,5-triazin

3,5 g (0,111 mol) Methylamin wurden bei 0 °C innerhalb 20 Minuten unter Rühren in eine Lösung von 11 g (0,055 Mol) 2-Amino-4-fluor-6-trifluormethoxy-1,3,5-triazin in 150 ml Tetrahydrofuran eingegast. Es wurde eine Stunde bei 0 °C und über Nacht bei 22 °C gerührt. Das Reaktionsgemisch wurde im Vakuum eingeengt, mit Wasser verrührt und getrocknet. Man erhielt 10,8 g (93,1 % d. Th.) der Titelverbindung vom Fp. 155 bis 157 °C (Zers.).

Beispiel II.6

2-Amino-4-chlordifluormethoxy-6-methylamino-1,3,5-triazin

2,9 g (0,093 mol) Methylamin wurden innerhalb 20 Minuten unter Rühren bei 0 °C in eine Lösung von 10 g (0,047 mol) 2-Amino-4-chlordifluormethoxy-6-fluor-1,3,5-triazin in 150 ml Diethylether eingegast. Es wurde eine Stunde bei 0 °C und über Nacht bei 22 °C gerührt. Nach dem Waschen mit Wasser, Trocknen und Einengen erhielt man 9,4 g (89,5 % d. Th.) der Titelverbindung vom Fp. 143 °C (Zers.).

Beispiel II.7

2-Amino-4-dimethylamino-6-trifluormethoxy-1,3,5-triazin

5,0 g (0,111 mol) Dimethylamin wurden innerhalb 20 Minuten unter Rühren bei 0 °C in eine Lösung von 11 g (0,055 mol) 2-Amino-4-fluor-6-trifluormethoxy-1,3,5-triazin in 150 ml Tetrahydrofuran eingegast. Es wurde eine Stunde bei 0 °C und über Nacht bei 22 °C gerührt. Nach dem Einengen, Waschen mit Wasser

und Trocknen erhielt man 9,9 g (80,7 % d. Th.) der Titelverbindung vom Fp. 114 bis 118 °C (Zers.).

Beispiel II.8

2-Amino-4-chlordifluormethoxy-6-dimethylamino-1,3,5-triazin

4,2 g (0,093 mol) Dimethylamin wurden innerhalb 20 Minuten unter Rühren bei 0 °C in eine Lösung von 10 g (0,047 mol) 2-Amino-4-chlordifluormethoxy-6-fluor-1,3,5-triazin in 150 ml Diethylether eingeleitet. Es wurde eine Stunde bei 0 °C und über Nacht bei 22 °C gerührt. Nach dem Waschen mit Wasser, Trocknen und Einengen erhielt man 9,8 g (87,8 % d. Th.) der Titelverbindung vom Fp. 130 bis 133 °C (Zers.).

III Herstellung der Sulfonylharnstoffverbindungen I

Beispiel III.1

2-(((4-Methoxy-6-trifluormethoxy-1,3,5-triazin-2-yl)aminocarbonyl)-aminosulfonyl)-benzoesäuremethylester

3,6 g (0,015 mol) 2-Carbomethoxy-benzosulfonylisocyanat in 4 ml 1,2-Dichlorethan wurden innerhalb 5 Minuten unter Rühren bei 22 °C zu einer Mischung 3,15 g (0,015 mol) 2-Amino-4-methoxy-6-trifluormethoxy-1,3,5-triazin in 150 ml 1,2-Dichlorethan gegeben und 12 Stunden bei 22 °C gerührt. Das Reaktionsgemisch wurde im Vakuum eingeengt und mit Methyl-tert.-butylether/Petrolether 1 : 1 kristallisiert, abgesaugt und mit Petrolether gewaschen. Man erhielt 5,1 g (75,4 % d. Th.) der Titelverbindung vom Fp. 149 °C (Zers.). (Wirkstoffbeispiel 5.001).

Beispiel III.2

2-(((4-Methoxy-6-trifluormethoxy-1,3,5-triazin-2-yl)aminocarbonyl)-aminosulfonyl)-benzoesäuremethylesternatriumsalz

1,8 g (0,004 mol) der Verbindung aus Beispiel III.1 wurden in 30 ml Methanol suspendiert und mit 0,72 g (0,004 Mol) 30%iger Natriummethylatlösung unter Rühren bei 10 bis 15 °C versetzt. Die klare Lösung wurde nach 10 Minuten Rühren im Vakuum eingeengt, wobei 1,9 g (100 % d. Th.) der Titelverbindung vom Fp. 118 °C (Zers.) anfielen. (Wirkstoffbeispiel 5.019).

Beispiel III.3

2-(((4-Methylamino-6-trifluormethoxy-1,3,5-triazin-2-yl)amino-carbonyl)-aminosulfonyl)-benzoesäureethylester

3,1 g (0,012 mol) 2-Carboethoxy-benzosulfonylisocyanat in 3 ml Methylenchlorid wurden innerhalb 10 Minuten unter Rühren bei 22 °C zu einer Mischung 2,5 g (0,012 mol) 2-Amino-4-methylamino-6-trifluormethoxy-1,3,5-triazin in 150 ml Methylenchlorid gegeben und 30 Stunden bei 22 °C gerührt. Das Reaktionsgemisch wurde im Vakuum eingeengt, mit Methyl-tert.-butylether verrührt und abgesaugt. Nach weiterem Waschen mit Methanol und Trocknen erhielt man 3,8 g (67,4 % d. Th.) der Titelverbindung vom Fp. 182 bis 184 °C (Zers.).
(Wirkstoffbeispiel 7.003).

Die in den nachstehenden Beispielen wiedergegeben Vorschriften wurden unter entsprechender Abwandlung der Ausgangsverbindungen zur Gewinnung weiterer Verbindungen der Formel I benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellen und untersuchten Verbindungen eine gleichartige Wirkung erwarten.

Tabelle 1

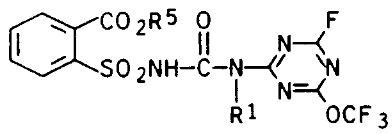

| Nr. | $R^1$ | $R^5$ | Fp (°C) | |
|---|---|---|---|---|
| 1.001 | H | $CH_3$ | 163 | |
| 1.002 | $CH_3$ | $CH_3$ | | |
| 1.003 | H | $CH_2CH_3$ | | |
| 1.004 | $CH_3$ | $CH_2CH_3$ | | |
| 1.005 | H | $(CH_2)_2CH_3$ | | |
| 1.006 | $CH_3$ | $(CH_2)_2CH_3$ | | |
| 1.007 | H | $CH(CH_3)_2$ | | |
| 1.008 | H | $CH_2-CH=CH_2$ | | |
| 1.009 | H | $CH_2-CH=CH-CH_3$ | | |
| 1.010 | H | $CH_2-C{\equiv}C-CH_3$ | | |
| 1.011 | H | $(CH_2)_2Cl$ | | |
| 1.012 | $CH_3$ | $(CH_2)_2Cl$ | | |
| 1.013 | H | $(CH_2)_2OCH_3$ | | |
| 1.014 | H | $(CH_2)_2O(CH_2)_2OCH_3$ | | |
| 1.015 | H | Cyclopentyl | | |
| 1.016 | H | Cyclohexyl | | |
| 1.017 | H | $CH_2CF_3$ | | |
| 1.018 | H | $(CH_2)_2SCH_3$ | | |
| 1.019 | H | $CH_3$ | | Na-salz |
| 1.020 | $CH_3$ | $CH_3$ | | Na-salz |
| 1.021 | H | $CH_2CH_3$ | | Na-salz |
| 1.022 | $CH_3$ | $CH_2CH_3$ | | Na-salz |
| 1.023 | H | $(CH_2)_2CH_3$ | | Na-salz |
| 1.024 | H | $(CH_2)_2Cl$ | | Na-salz |

Tabelle 2

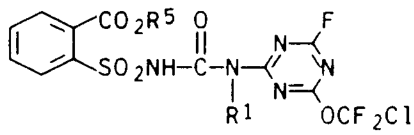

| Nr. | $R^1$ | $R^5$ | Fp ($^oC$) |
|---|---|---|---|
| 2.001 | H | $CH_3$ | |
| 2.002 | $CH_3$ | $CH_3$ | |
| 2.003 | H | $CH_2CH_3$ | |
| 2.004 | $CH_3$ | $CH_2CH_3$ | |
| 2.005 | H | $(CH_2)_2CH_3$ | |
| 2.006 | $CH_3$ | $(CH_2)_2CH_3$ | |
| 2.007 | H | $CH(CH_3)_2$ | |
| 2.008 | H | $CH_2-CH=CH_2$ | |
| 2.009 | H | $CH_2-CH=CH-CH_3$ | |
| 2.010 | H | $CH_2-C\equiv C-CH_3$ | |
| 2.011 | H | $(CH_2)_2Cl$ | |
| 2.012 | $CH_3$ | $(CH_2)_2Cl$ | |
| 2.013 | H | $(CH_2)_2OCH_3$ | |
| 2.014 | H | $(CH_2)_2O(CH_2)_2OCH_3$ | |
| 2.015 | H | Cyclopentyl | |
| 2.016 | H | Cyclohexyl | |
| 2.017 | H | $CH_2CF_3$ | |
| 2.018 | H | $(CH_2)_2SCH_3$ | |
| 2.019 | H | $CH_3$ | Na-salz |
| 2.020 | $CH_3$ | $CH_3$ | Na-salz |
| 2.021 | H | $CH_2CH_3$ | Na-salz |
| 2.022 | $CH_3$ | $CH_2CH_3$ | Na-salz |
| 2.023 | H | $(CH_2)_2CH_3$ | Na-salz |
| 2.024 | H | $(CH_2)_2Cl$ | Na-salz |

Tabelle 3

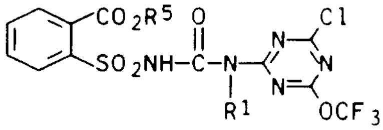

| Nr. | $R^1$ | $R^5$ | Fp (°C) |
|---|---|---|---|
| 3.001 | H | $CH_3$ | |
| 3.002 | $CH_3$ | $CH_3$ | |
| 3.003 | H | $CH_2CH_3$ | |
| 3.004 | $CH_3$ | $CH_2CH_3$ | |
| 3.005 | H | $(CH_2)_2CH_3$ | |
| 3.006 | $CH_3$ | $(CH_2)_2CH_3$ | |
| 3.007 | H | $CH(CH_3)_2$ | |
| 3.008 | H | $CH_2-CH=CH_2$ | |
| 3.009 | H | $CH_2-CH=CH-CH_3$ | |
| 3.010 | H | $CH_2-C\equiv C-CH_3$ | |
| 3.011 | H | $(CH_2)_2Cl$ | |
| 3.012 | $CH_3$ | $(CH_2)_2Cl$ | |
| 3.013 | H | $(CH_2)_2OCH_3$ | |
| 3.014 | H | $(CH_2)_2O(CH_2)_2OCH_3$ | |
| 3.015 | H | Cyclopentyl | |
| 3.016 | H | Cyclohexyl | |
| 3.017 | H | $CH_2CF_3$ | |
| 3.018 | H | $(CH_2)_2SCH_3$ | |
| 3.019 | H | $CH_3$ | Na-salz |
| 3.020 | $CH_3$ | $CH_3$ | Na-salz |
| 3.021 | H | $CH_2CH_3$ | Na-salz |
| 3.022 | $CH_3$ | $CH_2CH_3$ | Na-salz |
| 3.023 | H | $(CH_2)_2CH_3$ | Na-salz |
| 3.024 | H | $(CH_2)_2Cl$ | Na-salz |

Tabelle 4

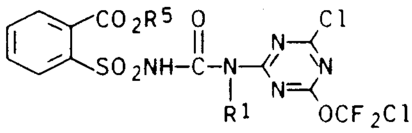

| Nr. | $R^1$ | $R^5$ | Fp (°C) |
|---|---|---|---|
| 4.001 | H | $CH_3$ | |
| 4.002 | $CH_3$ | $CH_3$ | |
| 4.003 | H | $CH_2CH_3$ | |
| 4.004 | $CH_3$ | $CH_2CH_3$ | |
| 4.005 | H | $(CH_2)_2CH_3$ | |
| 4.006 | $CH_3$ | $(CH_2)_2CH_3$ | |
| 4.007 | H | $CH(CH_3)_2$ | |
| 4.008 | H | $CH_2-CH=CH_2$ | |
| 4.009 | H | $CH_2-CH=CH-CH_3$ | |
| 4.010 | H | $CH_2-C\equiv C-CH_3$ | |
| 4.011 | H | $(CH_2)_2Cl$ | |
| 4.012 | $CH_3$ | $(CH_2)_2Cl$ | |
| 4.013 | H | $(CH_2)_2OCH_3$ | |
| 4.014 | H | $(CH_2)_2O(CH_2)_2OCH_3$ | |
| 4.015 | H | Cyclopentyl | |
| 4.016 | H | Cyclohexyl | |
| 4.017 | H | $CH_2CF_3$ | |
| 4.018 | H | $(CH_2)_2SCH_3$ | |
| 4.019 | H | $CH_3$ | Na-salz |
| 4.020 | $CH_3$ | $CH_3$ | Na-salz |
| 4.021 | H | $CH_2CH_3$ | Na-salz |
| 4.022 | $CH_3$ | $CH_2CH_3$ | Na-salz |
| 4.023 | H | $(CH_2)_2CH_3$ | Na-salz |
| 4.024 | H | $(CH_2)_2Cl$ | Na-salz |

Tabelle 5

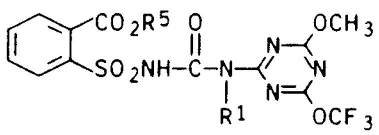

| Nr. | $R^1$ | $R^5$ | Fp ($^oC$) | |
|---|---|---|---|---|
| 5.001 | H | $CH_3$ | 149 Zers. | |
| 5.002 | $CH_3$ | $CH_3$ | | |
| 5.003 | H | $CH_2CH_3$ | | |
| 5.004 | $CH_3$ | $CH_2CH_3$ | | |
| 5.005 | H | $(CH_2)_2CH_3$ | | |
| 5.006 | $CH_3$ | $(CH_2)_2CH_3$ | | |
| 5.007 | H | $CH(CH_3)_2$ | | |
| 5.008 | H | $CH_2-CH=CH_2$ | | |
| 5.009 | H | $CH_2-CH=CH-CH_3$ | | |
| 5.010 | H | $CH_2-C\equiv C-CH_3$ | | |
| 5.011 | H | $(CH_2)_2Cl$ | | |
| 5.012 | $CH_3$ | $(CH_2)_2Cl$ | | |
| 5.013 | H | $(CH_2)_2OCH_3$ | | |
| 5.014 | H | $(CH_2)_2O(CH_2)_2OCH_3$ | | |
| 5.015 | H | Cyclopentyl | | |
| 5.016 | H | Cyclohexyl | | |
| 5.017 | H | $CH_2CF_3$ | | |
| 5.018 | H | $(CH_2)_2SCH_3$ | | |
| 5.019 | H | $CH_3$ | 118 Zers. | Na-salz |
| 5.020 | $CH_3$ | $CH_3$ | | Na-salz |
| 5.021 | H | $CH_2CH_3$ | | Na-salz |
| 5.022 | $CH_3$ | $CH_2CH_3$ | | Na-salz |
| 5.023 | H | $(CH_2)_2CH_3$ | | Na-salz |
| 5.024 | H | $(CH_2)_2Cl$ | | Na-salz |

Tabelle 6

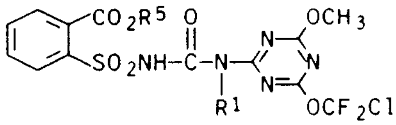

| Nr. | $R^1$ | $R^5$ | Fp (°C) | |
|---|---|---|---|---|
| 6.001 | H | $CH_3$ | 128-135 | |
| 6.002 | $CH_3$ | $CH_3$ | | |
| 6.003 | H | $CH_2CH_3$ | | |
| 6.004 | $CH_3$ | $CH_2CH_3$ | | |
| 6.005 | H | $(CH_2)_2CH_3$ | | |
| 6.006 | $CH_3$ | $(CH_2)_2CH_3$ | | |
| 6.007 | H | $CH(CH_3)_2$ | | |
| 6.008 | H | $CH_2$-CH=$CH_2$ | | |
| 6.009 | H | $CH_2$-CH=CH-$CH_3$ | | |
| 6.010 | H | $CH_2$-C≡C-$CH_3$ | | |
| 6.011 | H | $(CH_2)_2Cl$ | | |
| 6.012 | $CH_3$ | $(CH_2)_2Cl$ | | |
| 6.013 | H | $(CH_2)_2OCH_3$ | | |
| 6.014 | H | $(CH_2)_2O(CH_2)_2OCH_3$ | | |
| 6.015 | H | Cyclopentyl | | |
| 6.016 | H | Cyclohexyl | | |
| 6.017 | H | $CH_2CF_3$ | | |
| 6.018 | H | $(CH_2)_2SCH_3$ | | |
| 6.019 | H | $CH_3$ | 135 Zers. | Na-salz |
| 6.020 | $CH_3$ | $CH_3$ | | Na-salz |
| 6.021 | H | $CH_2CH_3$ | | Na-salz |
| 6.022 | $CH_3$ | $CH_2CH_3$ | | Na-salz |
| 6.023 | H | $(CH_2)_2CH_3$ | | Na-salz |
| 6.024 | H | $(CH_2)_2Cl$ | | Na-salz |

Tabelle 7

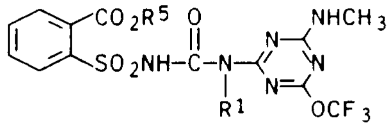

| Nr. | $R^1$ | $R^5$ | Fp (°C) |
|---|---|---|---|
| 7.001 | H | $CH_3$ | 162 Zers. |
| 7.002 | $CH_3$ | $CH_3$ | |
| 7.003 | H | $CH_2CH_3$ | 182-184 Zers. |
| 7.004 | $CH_3$ | $CH_2CH_3$ | |
| 7.005 | H | $(CH_2)_2CH_3$ | |
| 7.006 | $CH_3$ | $(CH_2)_2CH_3$ | |
| 7.007 | H | $CH(CH_3)_2$ | |
| 7.008 | H | $CH_2-CH=CH_2$ | |
| 7.009 | H | $CH_2-CH=CH-CH_3$ | |
| 7.010 | H | $CH_2-C\equiv C-CH_3$ | |
| 7.011 | H | $(CH_2)_2Cl$ | |
| 7.012 | $CH_3$ | $(CH_2)_2Cl$ | |
| 7.013 | H | $(CH_2)_2OCH_3$ | |
| 7.014 | H | $(CH_2)_2O(CH_2)_2OCH_3$ | |
| 7.015 | H | Cyclopentyl | |
| 7.016 | H | Cyclohexyl | |
| 7.017 | H | $CH_2CF_3$ | |
| 7.018 | H | $(CH_2)_2SCH_3$ | |
| 7.019 | H | $CH_3$ | 155-160 Zers. Na-salz |
| 7.020 | $CH_3$ | $CH_3$ | Na-salz |
| 7.021 | H | $CH_2CH_3$ | Na-salz |
| 7.022 | $CH_3$ | $CH_2CH_3$ | Na-salz |
| 7.023 | H | $(CH_2)_2CH_3$ | Na-salz |
| 7.024 | H | $(CH_2)_2Cl$ | Na-salz |

Tabelle 8

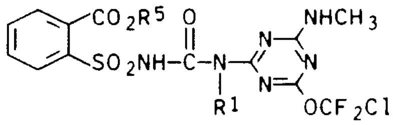

| Nr. | $R^1$ | $R^5$ | Fp (°C) | |
|---|---|---|---|---|
| 8.001 | H | $CH_3$ | 159 Zers. | |
| 8.002 | $CH_3$ | $CH_3$ | | |
| 8.003 | H | $CH_2CH_3$ | | |
| 8.004 | $CH_3$ | $CH_2CH_3$ | | |
| 8.005 | H | $(CH_2)_2CH_3$ | | |
| 8.006 | $CH_3$ | $(CH_2)_2CH_3$ | | |
| 8.007 | H | $CH(CH_3)_2$ | | |
| 8.008 | H | $CH_2-CH=CH_2$ | | |
| 8.009 | H | $CH_2-CH=CH-CH_3$ | | |
| 8.010 | H | $CH_2-C\equiv C-CH_3$ | | |
| 8.011 | H | $(CH_2)_2Cl$ | | |
| 8.012 | $CH_3$ | $(CH_2)_2Cl$ | | |
| 8.013 | H | $(CH_2)_2OCH_3$ | | |
| 8.014 | H | $(CH_2)_2O(CH_2)_2OCH_3$ | | |
| 8.015 | H | Cyclopentyl | | |
| 8.016 | H | Cyclohexyl | | |
| 8.017 | H | $CH_2CF_3$ | | |
| 8.018 | H | $(CH_2)_2SCH_3$ | | |
| 8.019 | H | $CH_3$ | 175-179 Zers. | Na-salz |
| 8.020 | $CH_3$ | $CH_3$ | | Na-salz |
| 8.021 | H | $CH_2CH_3$ | | Na-salz |
| 8.022 | $CH_3$ | $CH_2CH_3$ | | Na-salz |
| 8.023 | H | $(CH_2)_2CH_3$ | | Na-salz |
| 8.024 | H | $(CH_2)_2Cl$ | | Na-salz |

Tabelle 9

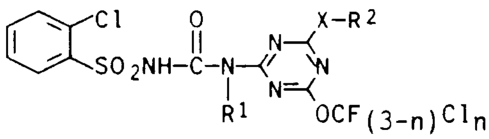

| Nr. | $R^1$ | X | $R^2$ | n | Fp (°C) |
|---|---|---|---|---|---|
| 9.001 | H | - | F | 0 | |
| 9.002 | H | - | Cl | 0 | |
| 9.003 | $CH_3$ | - | F | 0 | |
| 9.004 | $CH_3$ | - | Cl | 0 | |
| 9.005 | H | - | F | 1 | |
| 9.006 | H | - | Cl | 1 | |
| 9.007 | $CH_3$ | - | F | 1 | |
| 9.008 | $CH_3$ | - | Cl | 1 | |
| 9.009 | H | - | F | 0 | Na-salz |
| 9.010 | H | - | Cl | 0 | Na-salz |
| 9.011 | H | O | $CH_3$ | 0 | 165 (Zers.) |
| 9.012 | H | O | $CH_3$ | 1 | |
| 9.013 | $CH_3$ | O | $CH_3$ | 0 | |
| 9.014 | $CH_3$ | O | $CH_3$ | 1 | |
| 9.015 | $CH_3$ | O | $CH_3$ | 0 | Na-salz |
| 9.016 | $CH_3$ | O | $CH_3$ | 1 | Na-salz |
| 9.017 | H | NH | $CH_3$ | 0 | 149 (Zers.) |
| 9.018 | H | NH | $CH_3$ | 1 | |
| 9.019 | H | $NCH_3$ | $CH_3$ | 0 | 194 (Zers.) |
| 9.020 | H | $NCH_3$ | $CH_3$ | 1 | |
| 9.021 | $CH_3$ | $NCH_3$ | $CH_3$ | 0 | |
| 9.022 | H | O | $CH_3$ | 0 | Na-salz |
| 9.023 | H | $NCH_3$ | $CH_3$ | 0 | 168 Na-salz (Zersetzung) |

Tabelle 10

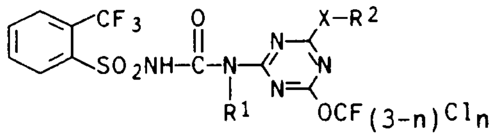

| Nr. | $R^1$ | X | $R^2$ | n | Fp (°C) | |
|---|---|---|---|---|---|---|
| 10.001 | H | - | F | 0 | | |
| 10.002 | H | - | Cl | 0 | | |
| 10.003 | H | - | F | 1 | | |
| 10.004 | H | - | Cl | 1 | | |
| 10.005 | H | O | $CH_3$ | 0 | | |
| 10.006 | H | O | $CH_3$ | 1 | 133 | |
| 10.007 | H | NH | $CH_3$ | 0 | 197 | |
| 10.008 | H | NH | $CH_3$ | 1 | | |
| 10.009 | $CH_3$ | NH | $CH_3$ | 0 | | |
| 10.010 | $CH_3$ | NH | $CH_3$ | 1 | | |
| 10.011 | H | O | $CH_3$ | 0 | 173 | Na-salz |
| 10.012 | H | $NCH_3$ | $CH_3$ | 0 | 174 | Na-salz |
| 10.013 | H | $NCH_3$ | $CH_3$ | 0 | 162 | |
| 10.014 | H | NH | $CH_3$ | 0 | 175 | Na-salz |

Tabelle 11

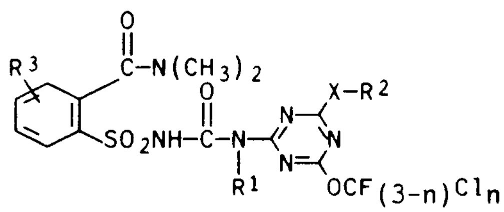

| Nr. | $R^1$ | X | $R^2$ | $R^3$ | n | Fp (°C) |
|---|---|---|---|---|---|---|
| 11.001 | H | - | F | - | 0 | |
| 11.002 | H | - | Cl | - | 0 | |
| 11.003 | H | - | F | - | 1 | |
| 11.004 | H | - | Cl | - | 1 | |
| 11.005 | H | O | $CH_3$ | - | 0 | |
| 11.006 | H | O | $CH_3$ | 3-F | 1 | |
| 11.007 | H | NH | $CH_3$ | - | 0 | |
| 11.008 | H | NH | $CH_3$ | - | 1 | |
| 11.009 | H | O | $CH_3$ | 5-Cl | 0 | |
| 11.010 | H | O | $CH_3$ | 5-Cl | 1 | |

Tabelle 12

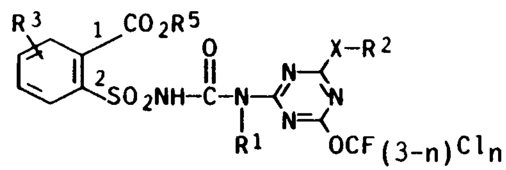

| Nr. | $R^1$ | X | $R^2$ | $R^3$ | $R^5$ | n | Fp (°C) |
|---|---|---|---|---|---|---|---|
| 12.001 | H | - | F | 3-F | $CH_3$ | 0 | |
| 12.002 | H | - | Cl | 3-F | $CH_3$ | 0 | |
| 12.003 | H | - | F | 3-F | $CH_3$ | 1 | |
| 12.004 | H | - | Cl | 3-F | $CH_3$ | 1 | |
| 12.005 | H | O | $CH_3$ | 3-F | $CH_3$ | 0 | |
| 12.006 | H | O | $CH_3$ | 3-F | $CH_3$ | 1 | |
| 12.007 | H | O | $CH_3$ | 5-Cl | $CH_3$ | 0 | |
| 12.008 | H | O | $CH_3$ | 5-Cl | $CH_3$ | 1 | |
| 12.009 | H | - | F | 5-Cl | $CH_3$ | 0 | |
| 12.010 | H | - | F | 5-Cl | $CH_3$ | 1 | |
| 12.011 | H | - | Cl | 5-Cl | $CH_3$ | 0 | |
| 12.012 | H | - | Cl | 5-Cl | $CH_3$ | 1 | |
| 12.013 | H | - | F | 6-$CH_3$ | $CH_3$ | 0 | |
| 12.014 | H | - | F | 6-$CH_3$ | $CH_3$ | 1 | |
| 12.015 | H | - | Cl | 6-$CH_3$ | $CH_3$ | 0 | |
| 12.016 | H | - | Cl | 6-$CH_3$ | $CH_3$ | 1 | |
| 12.017 | H | O | $CH_3$ | 6-$CH_3$ | $CH_3$ | 0 | |

Tabelle 12 (Forts.)

| Nr. | $R^1$ | X | $R^2$ | $R^3$ | $R^5$ | n | Fp (°C) | |
|---|---|---|---|---|---|---|---|---|
| 12.018 | H | O | $CH_3$ | 6-$CH_3$ | $CH_3$ | 1 | | |
| 12.019 | H | NH | $CH_3$ | 6-$CH_3$ | $CH_3$ | 0 | | |
| 12.020 | H | NH | $CH_3$ | 6-$CH_3$ | $CH_3$ | 1 | | |
| 12.021 | $CH_3$ | NH | $CH_3$ | 6-$CH_3$ | $CH_3$ | 0 | | |
| 12.022 | H | NH | $CH_3$ | 6-$CH_3$ | $CH_3$ | 0 | | Na-salz |
| 12.023 | H | - | $CF_3$ | - | $CH_3$ | 0 | | |
| 12.024 | H | - | $CF_3$ | - | $CH_3$ | 0 | | |
| 12.025 | $CH_3$ | - | $CF_3$ | - | $CH_3$ | 0 | | |
| 12.026 | H | - | $CF_3$ | - | $CH_3$ | 1 | | |
| 12.027 | H | N-$CH_3$ | $CH_3$ | - | $CH_3$ | 0 | 172 Zers. | |
| 12.028 | H | N-$CH_3$ | $CH_3$ | - | $CH_3$ | 0 | 170-175 Zers. | Na-Salz |
| 12.029 | H | N-$CH_3$ | $CH_3$ | 6-$CH_3$ | $CH_3$ | 0 | | |
| 12.030 | H | N-$CH_3$ | $CH_3$ | 6-$CH_3$ | $CH_3$ | 1 | | |
| 12.031 | H | N-$CH_3$ | $CH_3$ | - | $CH_3$ | 0 | | |
| 12.032 | H | N-$CH_3$ | $CH_3$ | - | $CH_3$ | 1 | 158 Zers. | |
| 12.033 | H | O | $C_2H_5$ | - | $CH_3$ | 0 | 160-164 | |
| 12.034 | H | O | $C_2H_5$ | - | $CH_3$ | 0 | | Na-Salz |
| 12.035 | H | O | $C_2H_5$ | - | $CH_3$ | 1 | 83-86 Zers. | |
| 12.036 | $CH_3$ | O | $C_2H_5$ | - | $CH_3$ | 1 | | Na-Salz |
| 12.037 | H | O | $C_2H_5$ | 6-$CH_3$ | $CH_3$ | 0 | | |
| 12.038 | H | O | $C_2H_5$ | 5-Cl | $CH_3$ | 0 | | |
| 12.039 | $CH_3$ | O | $C_2H_5$ | 5-Cl | $CH_3$ | 0 | | |
| 12.040 | H | O | $C_2H_5$ | 5-Cl | $CH_3$ | 1 | | |

Tabelle 12 (Forts.)

| Nr. | $R^1$ | X | $R^2$ | $R^3$ | $R^5$ | n | Fp (°C) | |
|---|---|---|---|---|---|---|---|---|
| 12.041 | H | O | $CH_3$ | - | $C_2H_5$ | 0 | | |
| 12.042 | H | O | $CH_3$ | - | $C_2H_5$ | 0 | | Na-Salz |
| 12.043 | H | O | $CH_3$ | - | $C_2H_5$ | 1 | | |
| 12.044 | H | O | $C_2H_5$ | 5-Cl | $CH_3$ | 0 | | Na-Salz |
| 12.045 | H | O | $CH_3$ | 3-Cl | $CH_3$ | 0 | | |
| 12.046 | H | O | $CH_3$ | 3-Cl | $CH_3$ | 0 | | Na-Salz |
| 12.047 | H | O | $CH_3$ | 4-Cl | $CH_3$ | 0 | | |
| 12.048 | H | O | $CH_3$ | 4-Cl | $CH_3$ | 0 | | Na-Salz |
| 12.049 | H | O | $CH_3$ | 3-Cl | $CH_3$ | 1 | | |
| 12.050 | H | O | $CH_3$ | 3-Cl | $CH_3$ | 1 | | Na-Salz |
| 12.051 | H | NH | $CH_3$ | 3-Cl | $CH_3$ | 0 | | |
| 12.052 | H | NH | $CH_3$ | 3-Cl | $CH_3$ | 0 | | Na-Salz |
| 12.053 | H | NH | $CH_3$ | 3-Cl | $CH_3$ | 1 | | |
| 12.054 | H | NH | $CH_3$ | 3-Cl | $CH_3$ | 1 | | Na-Salz |
| 12.055 | H | $NCH_3$ | $CH_3$ | 3-Cl | $CH_3$ | 0 | | |
| 12.056 | H | $NCH_3$ | $CH_3$ | 3-Cl | $CH_3$ | 0 | | Na-Salz |
| 12.057 | H | $NCH_3$ | $CH_3$ | 3-Cl | $CH_3$ | 1 | | |
| 12.058 | H | $NCH_3$ | $CH_3$ | 3-Cl | $CH_3$ | 1 | | Na-Salz |
| 12.059 | $CH_3$ | $NCH_3$ | $CH_3$ | 3-Cl | $CH_3$ | 0 | | |
| 12.060 | H | - | F | 3-Cl | $CH_3$ | 0 | | |
| 12.061 | H | - | F | 3-Cl | $CH_3$ | 0 | | Na-Salz |
| 12.062 | H | - | F | 3-Cl | $CH_3$ | 1 | | |
| 12.063 | H | - | F | 3-Cl | $CH_3$ | 1 | | Na-Salz |

Tabelle 12 (Forts.)

| Nr. | $R^1$ | X | $R^2$ | $R^3$ | $R^5$ | n | Fp ($^oC$) | |
|---|---|---|---|---|---|---|---|---|
| 12.064 | H | - | Cl | 3-Cl | $CH_3$ | 0 | | |
| 12.065 | H | O | $CH_3$ | 3-$CH_3$ | $CH_3$ | 0 | | |
| 12.066 | H | O | $CH_3$ | 3-$CH_3$ | $CH_3$ | 0 | | Na-Salz |
| 12.067 | H | O | $CH_3$ | 3-$CH_3$ | $CH_3$ | 1 | | |
| 12.068 | H | O | $CH_3$ | 3-$CH_3$ | $CH_3$ | 1 | | Na-Salz |
| 12.069 | H | NH | $CH_3$ | 3-$CH_3$ | $CH_3$ | 0 | | |
| 12.070 | H | NH | $CH_3$ | 3-$CH_3$ | $CH_3$ | 0 | | Na-Salz |
| 12.071 | H | NH | $CH_3$ | 3-$CH_3$ | $CH_3$ | 1 | | |
| 12.072 | H | NH | $CH_3$ | 3-$CH_3$ | $CH_3$ | 1 | | Na-Salz |
| 12.073 | H | $NCH_3$ | $CH_3$ | 3-$CH_3$ | $CH_3$ | 0 | 128 Zers. | |
| 12.074 | H | $NCH_3$ | $CH_3$ | 3-$CH_3$ | $CH_3$ | 0 | | Na-Salz |
| 12.075 | H | $NCH_3$ | $CH_3$ | 3-$CH_3$ | $CH_3$ | 1 | | |
| 12.076 | H | $NCH_3$ | $CH_3$ | 3-$CH_3$ | $CH_3$ | 1 | | Na-Salz |
| 12.077 | H | - | F | 3-$CH_3$ | $CH_3$ | 0 | | |
| 12.078 | H | - | F | 3-$CH_3$ | $CH_3$ | 0 | | Na-Salz |
| 12.079 | H | - | F | 3-$CH_3$ | $CH_3$ | 1 | | |
| 12.080 | H | - | F | 3-$CH_3$ | $CH_3$ | 1 | | Na-Salz |
| 12.081 | H | - | Cl | 3-$CH_3$ | $CH_3$ | 0 | | |
| 12.082 | H | O | $CH_3$ | 4-Cl | $CH_3$ | 0 | | |
| 12.083 | H | O | $CH_3$ | 4-Cl | $CH_3$ | 0 | | Na-Salz |
| 12.084 | H | O | $CH_3$ | 4-Cl | $CH_3$ | 1 | | |
| 12.085 | H | O | $CH_3$ | 4-Cl | $CH_3$ | 1 | | Na-Salz |

Tabelle 12 (Forts.)

| Nr. | $R^1$ | X | $R^2$ | $R^3$ | $R^5$ | n | Fp (°C) |
|---|---|---|---|---|---|---|---|
| 12.086 | H | NH | $CH_3$ | 4-Cl | $CH_3$ | 0 | |
| 12.087 | H | NH | $CH_3$ | 4-Cl | $CH_3$ | 0 | Na-Salz |
| 12.088 | H | NH | $CH_3$ | 4-Cl | $CH_3$ | 1 | |
| 12.089 | H | NH | $CH_3$ | 4-Cl | $CH_3$ | 1 | Na-Salz |
| 12.090 | H | $NCH_3$ | $CH_3$ | 4-Cl | $CH_3$ | 0 | |
| 12.091 | H | $NCH_3$ | $CH_3$ | 4-Cl | $CH_3$ | 0 | Na-Salz |
| 12.092 | H | $NCH_3$ | $CH_3$ | 4-Cl | $CH_3$ | 1 | 100 Zers. |
| 12.093 | H | $NCH_3$ | $CH_3$ | 4-Cl | $CH_3$ | 1 | 165 Zers. Na-Salz |
| 12.094 | H | - | F | 4-Cl | $CH_3$ | 0 | |
| 12.095 | H | O | $C_2H_5$ | - | $CH_3$ | 1 | 139 Zers. Na-Salz |
| 12.096 | H | $NCH_3$ | $CH_3$ | - | $C_2H_5$ | 0 | 130-135 Zers. |
| 12.097 | H | $NCH_3$ | $CH_3$ | - | $CH_3$ | 1 | 175 Zers. Na-Salz |
| 12.098 | H | O | $CH_3$ | 4-$CH_3$ | $CH_3$ | 1 | |
| 12.099 | H | O | $CH_3$ | 4-$CH_3$ | $CH_3$ | 1 | |
| 12.100 | H | O | $CH_3$ | 4-$OCH_3$ | $CH_3$ | 1 | |
| 12.101 | H | NH | $CH_3$ | 4-$OCH_3$ | $CH_3$ | 1 | |
| 12.102 | H | $NCH_3$ | $CH_3$ | 4-$OCH_3$ | $CH_3$ | 1 | |
| 12.103 | H | O | $CH_3$ | 4-$OCH_3$ | $CH_3$ | 0 | |
| 12.104 | H | NH | $CH_3$ | 4-$OCH_3$ | $CH_3$ | 0 | |
| 12.105 | H | $NCH_3$ | $CH_3$ | 4-$OCH_3$ | $CH_3$ | 0 | |

Anwendungsbeispiele

A Herbizide Wirkung

Die herbizide Wirkung der Sulfonylharnstoffe der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und

Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmengen betrugen 0,125 kg/ha a.S.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,125 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürz. | Lateinischer Name | Deutscher Name |
|---|---|---|
| AMARE | Amaranthus retroflexus | Zurückgekrümmter Fuchsschwanz |
| GALAP | Galium aparine | Klettenlabkraut |
| POLPE | Polygonum persicaria | Flohknöterich |
| TRZAW | Triticum aestivum | Winterweizen |

Mit 0,125 kg/ha a.S. im Vor- wie Nachauflaufverfahren eingesetzt, lassen sich mit dem Beispiel 5.019 breitblättrige unerwünschte Pflanzen sehr gut bekämpfen bei gleichzeitiger Verträglichkeit an Weizen.

B Bioregulatorische Wirkung

B.1 Wachstumsregulierende Wirkung

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Nachauflaufverfahren wurden die zu prüfenden Substanzen (aktive Substanz, a.S.) in wäßriger Aufbereitung auf die Pflanzen gesprüht. Die beobachtete wachstumsregulierende Wirkung wurde bei versuchsende durch Wuchshöhenmessung belegt. Die so gewonnenen Meßwerte wurden zur Wuchshöhe der unbehandelten Pflanzen in Relation gesetzt. Als Vergleichssubstanz A diente 2-Chlorethyltrimethylammoniumchlorid (CCC).

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen.

Tabelle B.1.1

| Sommerweizen, "Ralle" Nachauflauf-Blattbehandlung | | |
|---|---|---|
| Nr. d. chem. Beispiele | Konzentration mg a.S./Gefäß | relative Wuchshöhen |
| unbehandelt | - | 100 |
| A | 0,38<br>1,5 | 91,4<br>86,3 |
| 12.032 | 0,38<br>1,5 | 67,3<br>45,7 |

Tabelle B.1.2

| Sommergerste, "Aramir" Nachauflauf-Blattbehandlung | | |
|---|---|---|
| Nr. d. chem. Beispiele | Konzentration mg a.S./Gefäß | relative Wuchshöhen |
| unbehandelt | - | 100 |
| A | 0,38<br>1,5 | 100<br>92,9 |
| 12.032 | 0,38<br>1,5 | 69,3<br>51,3 |

Tabelle B.1.3

| Sommerraps, "Petranova" Nachauflauf-Blattbehandlung | | |
|---|---|---|
| Nr. d. chem. Beispiele | Konzentration mg a.S./Gefäß | relative Wuchshöhen |
| unbehandelt | - | 100 |
| A | 0,025<br>0,1 | 99,4<br>94,3 |
| 12.032 | 0,025<br>0,1 | 72,4<br>60,6 |

B.2 Untersuchung auf Wirkung als Defolians in Baumwolle

Junge Baumwollpflanzen (Sorte Stoneville 825, Entwicklungsstadium: 5 - 6 entwickelte Laubblätter) wurden unter Gewächshausbedingungen angezogen (Tag/Nachttemperatur 25/18 °C, relative Luftfeuchte 50 - 70 %) und tropfnaß mit wäßrigen Aufbereitungen der angegebenen Wirkstoffe (unter Zusatz von 0,15 Gew.-% des Fettalkoholalkoxylats Plurafac® LF 700 bezogen auf die Spritzbrühe) blattbehandelt. Die umgerechnete Wassermenge betrug 1000 l/ha. 13 Tage nach Wirkstoffapplikation wurde die Anzahl abgeworfener Blätter bestimmt und der Grad der Entblätterung in % zur Kontrolle angegeben. Bei den unbehandelten Kontrollpflanzen trat kein Blattfall auf. Als Vergleichsmittel B diente 6,7-Dihydrodipyridol-(1,2-:2',1'-c)pyridilium-Ion als Dibromid-Monohydrat-Salz (Diquat). Die Ergebnisse sind in Tabelle B.2.1 zusammengestellt.

Tabelle B.2.1

| Mittel, enthaltend Wirkstoff Nr. | Umgerechnete Aufwandmenge a.S.[kg/ha] | Entblätterung [%] |
|---|---|---|
| 5.019 | 0,25<br>0,50 | 49<br>64 |
| 12.027 | 0,25<br>0,50 | 31<br>31 |
| Vergleichsmittel B | 0,5 | 36 |

Außerdem vermindern die erfindungsgemäßen Mittel den Wiederaustrieb der Pflanzen nach der Entblätterung. Ein solcher Effekt begünstigt die maschinelle Beerntung der Baumwollpflanzen.

## Patentansprüche

1. Substituierte Sulfonylharnstoffe der allgemeinen Formel I,

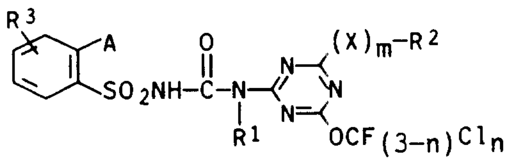

I,

in der n und m für 0 oder 1 stehen und die Substituenten folgende Bedeutung haben:
$R^1$
Wasserstoff, $C_1$-$C_4$-Alkyl, $C_3$-$C_6$-Alkenyl oder $C_3$-$C_6$-Alkinyl;
$R^2$
Halogen oder Trifluormethyl, wenn m für 0 steht oder $C_1$-$C_4$-Alkyl, $C_3$-$C_6$-Alkenyl oder $C_3$-$C_6$-Alkinyl, wenn m 1 bedeutet oder Trifluor- oder Chlordifluormethyl wenn X für 0 oder S und m für 1 steht;
X
O, S oder N-$R^4$, wobei $R^4$ für Wasserstoff oder $C_1$-$C_4$-Alkyl steht;
$R^3$
Wasserstoff, Halogen, $C_1$-$C_4$-Alkyl, $C_1$-$C_4$-Halogenalkyl oder $C_1$-$C_4$-Alkoxy;
A
$C_1$-$C_4$-Halogenalkyl, ein Halogenatom, CN, $NO_2$, $C_1$-$C_4$-Alkoxy, $C_1$-$C_4$-Alkylthio; $C_1$-$C_4$-Alkylsulfinyl- oder -sulfonyl oder ein Rest,

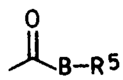

wobei
B
ein Sauerstoffatom oder eine Alkyliminogruppe N-$R^6$;
$R^5$
Wasserstoff, eine $C_1$-$C_6$-Alkylgruppe, welche bis zu drei der folgenden Reste tragen kann: Halogen, $C_1$-$C_4$-Alkoxy, $C_1$-$C_4$-Alkylthio, $C_1$-$C_4$-Halogenalkoxy, $C_2$-$C_4$-Alkoxy-$C_1$-$C_2$-alkoxy, $C_3$-$C_7$-Cycloalkyl und/oder Phenyl; eine $C_5$-$C_7$-Cycloalkylgruppe, welche bis zu drei $C_1$-$C_4$-Alkylgruppen tragen kann; eine $C_3$-$C_6$-Alkenylgruppe oder eine $C_3$-$C_6$-Alkinylgruppe;
$R^6$
Wasserstoff, eine $C_1$-$C_6$-Alkylgruppe, oder gemeinsam mit $R^5$ eine $C_4$-$C_6$-Alkylenkette, worin eine Methylengruppe durch ein Sauerstoffatom oder eine $C_1$-$C_4$-Alkyliminogruppe ersetzt sein kann, bedeutet,
sowie deren umweltverträgliche Salze.

2. Sulfonylharnstoffe nach Anspruch 1, in denen die Substituenten folgende Bedeutung haben:
$R^1$
Wasserstoff oder Methyl;
$R^2$
Methyl, wenn m 1 bedeutet;
X
O oder NH;
$R^3$
Wasserstoff, Halogen oder Methyl und
A
eine Gruppe $CO_2R^5$, worin $R^5$ $C_1$-$C_4$-Alkyl bedeutet;

3. Herbizides Mittel, enthaltend einen Sulfonylharnstoff der Formel I gemäß Anspruch 1 oder sein Salz sowie hierfür übliche Trägerstoffe.

4. Verfahren zur Bekämpfung unerwünschten Pflanzenwuchses, dadurch gekennzeichnet, daß man einen Sulfonylharnstoff der Formel I gemäß Anspruch oder eines seiner Salze in einer herbizid wirksamen Menge auf die Pflanzen und/oder ihren Lebensraum einwirken läßt.

5. Verfahren zur Bekämpfung unerwünschten Pflanzenwachstums in Getreide, dadurch gekennzeichnet, daß man eine herbizid wirksame Menge von 2-(((4-Methoxy-6-trifluormethoxy-1,3,5-triazin-2-yl)-aminocarbonyl)-aminosulfonyl)-benzoesäuremethylester verwendet.

6. Verfahren zur Regulierung des Pflanzenwuchses, dadurch gekennzeichnet, daß man eine wachstumsregulierende Menge eines Sulfonylharnstoffes der Formel I gemäß Anspruch 1 oder eines seiner Salze auf die Samen, die Pflanzen und/oder deren Lebensraum einwirken läßt.

7. Verfahren zur Herstellung von Sulfonylharnstoffen der Formel I gemäß Anspruch 1, in der A nicht die Bedeutung COOH besitzt, dadurch gekennzeichnet, daß man ein Sulfonylisocyanat II

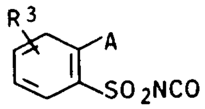

II

in an sich bekannter Weise in einem inerten organischen Lösungsmittel mit der ungefähr stöchiometrischen Menge eines substituierten 2-Amino-4-fluoralkoxy-1,3,5-triazins der Formel III

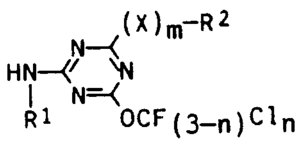

III

umsetzt.

8. Verfahren zur Herstellung von Sulfonylharnstoffen der Formel I gemäß Anspruch 7, dadurch gekennzeichnet, daß man ein Carbamat der Formel IV

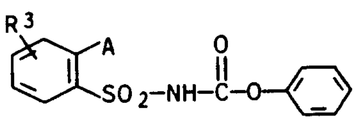

IV

in an sich bekannter Weise in einem inerten organischen Lösungsmittel bei einer Temperatur zwischen 0 und 120 °C mit ungefähr der stöchiometrischen Menge eines substituierten 2-Amino-4-fluoralkoxy-1,3,5-triazins III umsetzt.

9. Verfahren zur Herstellung von Sulfonylharnstoffen der Formel I gemäß Anspruch 7, dadurch gekennzeichnet, daß man ein entsprechendes Sulfonamid der Formel V

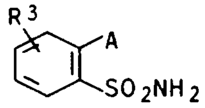

V

in an sich bekannter Weise in einem inerten organischen Lösungsmittel mit einem Phenylcarbamat VI

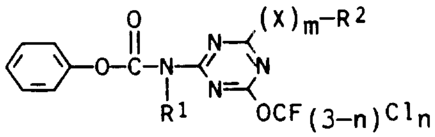

VI

umsetzt.

**10.** Substituierte 2-Amino-4-fluoralkoxy-1,3,5-triazine der allgemeinen Formel IIIa

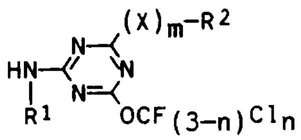

IIIa

in der m für 1 und n für 0 oder 1 steht und die Substituenten folgende Bedeutung haben:
$R^1$
Wasserstoff, $C_1$-$C_4$-Alkyl, $C_3$-$C_6$-Alkenyl oder $C_3$-$C_6$-Alkinyl;
$R^2$
$C_1$-$C_4$-Alkyl, $C_3$-$C_6$-Alkenyl oder $C_3$-$C_6$-Alkinyl;
X
O, S oder N-$R^4$, wobei
$R^4$
für Wasserstoff oder $C_1$-$C_4$-Alkyl steht.

**11.** Verfahren zur Herstellung von 2-Amino-4-fluoralkoxy-1,3,5-triazinen der allgemeinen Formel IIIa gemäß Anspruch 10, dadurch gekennzeichnet, daß man 2-Amino-4-fluoralkoxy-6-halogen-1,3,5-triazine der Formel IIIb

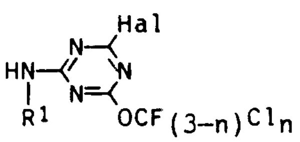

IIIb

in der Hal für Fluor, Chlor oder Brom steht und $R^1$ und n die vorgenannte Bedeutung haben, mit einem Nucleophil der Formel XVI

H-X-$R^2$ XVI

in der X und $R^2$ die vorgenannte Bedeutung besitzen, oder dessen Salz umsetzt.

**12.** 2-Amino-4-methoxy-6-trifluormethoxy-1,3,5-triazin.

**13.** 2-Amino-4-methoxy-6-chlordifluormethoxy-1,3,5-triazin.

**14.** 2-Amino-4-methylamino-6-trifluormethoxy-1,3,5-triazin.

**15.** 2-Amino-4-methylamino-6-chlordifluormethoxy-1,3,5-triazin.

**16.** 2-Amino-4-dimethylamino-6-trifluormethoxy-1,3,5-triazin.

**17.** 2-Amino-4-chlordifluormethoxy-6-dimethylamino-1,3,5-triazin.

**18.** 2-Amino-4-ethoxy-6-trifluormethoxy-1,3,5-triazin.

**19.** 2-Amino-4-chlordifluormethoxy-6-ethoxy-1,3,5-triazin.

**20.** 2,4-Bismethylamino-6-trifluormethoxy-1,3,5-triazin.

## Claims

**1.** A substituted sulfonylurea of the formula I

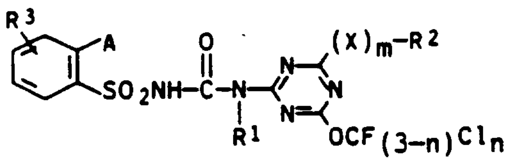

I

where n and m are each 0 or 1;
$R^1$ is hydrogen, $C_1$-$C_4$-alkyl, $C_3$-$C_6$-alkenyl or $C_3$-$C_6$-alkynyl;
$R^2$ is halogen or trifluoromethyl when m is 0, or $C_1$-$C_4$-alkyl, $C_3$-$C_6$-alkenyl or $C_3$-$C_6$-alkynyl when m is 1, or trifluoromethyl or chlorodifluoromethyl when X is O or S and m is 1;
X is O, S or N-$R^4$, where $R^4$ is hydrogen or $C_1$-$C_4$-alkyl;
$R^3$ is hydrogen, halogen, $C_1$-$C_4$-alkyl, $C_1$-$C_4$-haloalkyl or $C_1$-$C_4$-alkoxy;
A is $C_1$-$C_4$-haloalkyl, halogen, CN, $NO_2$, $C_1$-$C_4$-alkoxy, $C_1$-$C_4$-alkylthio, $C_1$-$C_4$-alkylsulfinyl or -sulfonyl or a radical

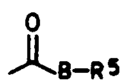

B is oxygen or an alkylimino group N-$R^6$;
$R^5$ is hydrogen, $C_1$-$C_6$-alkyl which may carry up to three of the following radicals: halogen, $C_1$-$C_4$-alkoxy, $C_1$-$C_4$-alkylthio, $C_1$-$C_4$-haloalkoxy, $C_2$-$C_4$-alkoxy-$C_1$- or -$C_2$-alkoxy, $C_3$-$C_7$-cycloalkyl and/or phenyl; $C_5$-$C_7$-cycloalkyl which may carry up to three $C_1$-$C_4$-alkyl groups; $C_3$-$C_6$-alkenyl or $C_3$-$C_6$-alkynyl, and
$R^6$ is hydrogen or $C_1$-$C_6$-alkyl or, together with $R^5$, forms a $C_4$-$C_6$-alkylene chain in which a methylene group may be replaced by an oxygen atom or a $C_1$-$C_4$-alkylimino group, and its environmentally tolerated salts.

**2.** A sulfonylurea as claimed in claim 1, where
$R^1$ is hydrogen or methyl,
$R^2$ is methyl when m is 1,
X is O or NH,
$R^3$ is hydrogen, halogen or methyl and
A is $CO_2R^5$, where $R^5$ is $C_1$-$C_4$-alkyl.

**3.** A herbicide containing a sulfonylurea of the formula I as claimed in claim 1 or its salt and conventional carriers.

**4.** A method for controlling undesirable plant growth, wherein a sulfonylurea of the formula I as claimed in claim 1 or one of its salts is allowed to act, in a herbicidal amount, on the plants and/or their habitat.

**5.** A method for controlling undesirable plant growth in cereals, wherein a herbicidal amount of methyl 2-(-((4-methoxy-6-trifluoromethoxy-1,3,5-triazin-2-yl)-aminocarbonyl)-aminosulfonyl)-benzoate is used.

**6.** A method for regulating plant growth, wherein an amount, having a growth-regulating effect, of a sulfonylurea of the formula I as claimed in claim 1 or one of its salts is allowed to act on the seeds, the plants and/or their habitat.

**7.** A process for the preparation of a sulfonylurea of the formula I as claimed in claim 1, where A is not COOH, wherein a sulfonyl isocyanate II

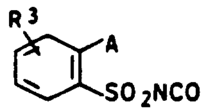

II

is reacted with about the stoichiometric amount of a substituted 2-amino-4-fluoroalkoxy-1,3,5-triazine of the formula III

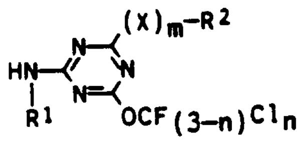

III

in a conventional manner in an inert organic solvent.

**8.** A process for the preparation of a sulfonylurea of the formula I as claimed in claim 7, wherein a carbamate of the formula IV

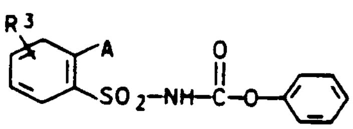

IV

is reacted with about the stoichiometric amount of a substituted 2-amino-4-fluoroalkoxy-1,3,5-triazine III in a conventional manner in an inert organic solvent at from 0 to 120 ° C.

**9.** A process for the preparation of a sulfonylurea of the formula I as claimed in claim 7, wherein a corresponding sulfonamide of the formula V

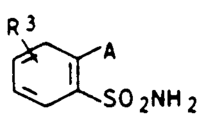

V

is reacted with a phenyl carbamate VI

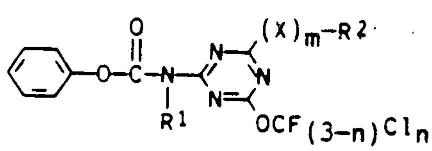

VI

in a conventional manner in an inert organic solvent.

**10.** A substituted 2-amino-4-fluoroalkoxy-1,3,5-triazine of the formula IIIa

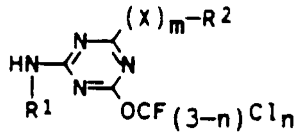

IIIa

where m is 1 and n is 0 or 1,
$R^1$ is hydrogen, $C_1$-$C_4$-alkyl, $C_3$-$C_6$-alkenyl or $C_3$-$C_6$-alkynyl,
$R^2$ is $C_1$-$C_4$-alkyl, $C_3$-$C_6$-alkenyl or $C_3$-$C_6$-alkynyl,
X is O, S or N-$R^4$ and
$R^4$ is hydrogen or $C_1$-$C_4$-alkyl.

**11.** A process for the preparation of a 2-amino-4-fluoroalkoxy-1,3,5-triazine of the formula IIIa as claimed in claim 10, wherein a 2-amino-4-fluoroalkoxy-6-halo-1,3,5-triazine of the formula IIIb

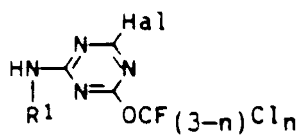

IIIb

where Hal is fluorine, chlorine or bromine and $R^1$ and n have the abovementioned meanings, is reacted with a nucleophile of the formula XVI

H-X-$R^2$ XVI,

where X and $R^2$ have the abovementioned meanings, or its salt.

**12.** 2-Amino-4-methoxy-6-trifluoromethoxy-1,3,5-triazine.

**13.** 2-Amino-4-methoxy-6-chlorodifluoromethoxy-1,3,5-triazine.

**14.** 2-Amino-4-methylamino-6-trifluoromethoxy-1,3,5-triazine.

**15.** 2-Amino-4-methylamino-6-chlorodifluoromethoxy-1,3,5-triazine.

**16.** 2-Amino-4-dimethylamino-6-trifluoromethoxy-1,3,5-triazine.

**17.** 2-Amino-4-chlorodifluoromethoxy-6-dimethylamino-1,3,5-triazine.

**18.** 2-Amino-4-ethoxy-6-trifluoromethoxy-1,3,5-triazine.

**19.** 2-Amino-4-chlorodifluoromethoxy-6-ethoxy-1,3,5-triazine.

**20.** 2,4-Bismethylmino-6-trifluoromethoxy-1,3,5-triazine.

## Revendications

1. Sulfonylurées substituées de formule générale I

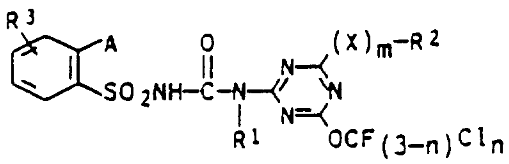

I,

dans laquelle n et m sont égaux à 0 ou 1 et les symboles ont les significations suivantes :

$R^1$
l'hydrogène, un groupe alkyle en C1-C4, alcényle en C3-C6 ou alcynyle en C3-C6 ;
$R^2$
un halogène ou un groupe trifluorométhyle lorsque m est égal à 0 ou un groupe alkyle en C1-C4, alcényle en C3-C6 ou alcynyle en C3-C6 lorsque m est égal à 1, ou bien un groupe trifluoro- ou chlorodifluorométhyle lorsque X représente O ou S et que m est égal à 1 ;
X
O, S ou N-$R^4$ dans lequel $R^4$ représente l'hydrogène ou un groupe alkyle en C1-C4 ;
$R^3$
l'hydrogène, un halogène, un groupe alkyle en C1-C4, halogénoalkyle en C1-C4 ou alcoxy en C1-C4 ;
A
un groupe halogénoalkyle en C1-C4, un atome d'halogène, un groupe CN, $NO_2$, alcoxy en C1-C4, alkylthio en C1-C4 ; alkylsulfinyle ou alkylsulfonyle en C1-C4, ou un groupe

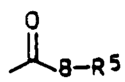

dans lequel
B
représente un atome d'oxygène ou un groupe alkylimino N-$R^6$ ;
$R^5$
l'hydrogène, un groupe alkyle en C1-C6 qui peut porter un à trois des substituants suivants : les halogènes, les groupes alcoxy en C1-C4, alkylthio en C1-C4, halogénoalcoxy en C1-C4, (alcoxy en C2-C4)-alcoxy en C1-C2, cycloalkyle en C3-C7 et/ou phényle ; un groupe cycloalkyle en C5-C7 qui peut porter jusqu'à trois groupes alkyle en C1-C4 ; un groupe alcényle en C3-C6 ou alcynyle en C3-C6 ;
$R^6$
l'hydrogène, un groupe alkyle en C1-C6, ou bien $R^6$ et $R^5$ forment ensemble une chaîne alkylène en C4-C6 dans laquelle un groupe méthylène peut être remplacé par un atome d'oxygène ou par un groupe alkylimino en C1-C4,
et leurs sels non polluants.

2. Sulfonylurées selon la revendication 1, pour lesquelles les symboles ont les significations suivantes :

$R^1$
l'hydrogène ou un groupe méthyle ;
$R^2$
un groupe méthyle lorsque m = 1 ;
X
O ou NH ;
$R^3$
l'hydrogène, un halogène ou un groupe méthyle et
A
un groupe $CO_2R^5$ dans lequel $R^5$ représente un groupe alkyle en C1-C4.

3. Produit herbicide contenant une sulfonylurée de formule I de la revendication 1 ou l'un de ses sels avec des véhicules usuels.

4. Procédé pour combattre les croissances de végétaux indésirables, caractérisé en ce que l'on fait agir sur les végétaux et/ou leur habitat une sulfonylurée de formule I de la revendication 1 ou l'un de ses sels en quantité herbicide efficace.

5. Procédé pour combattre les croissances de végétaux indésirables dans les cultures de céréales, caractérisé en ce que l'on utilise une quantité herbicide efficace de 2-(((4-méthoxy-6-trifluorométhoxy-1,3,5-triazine-2-yl)-aminocarbonyl)-aminosulfonyl)-benzoate de méthyle.

6. Procédé pour la régulation de la croissance des végétaux, caractérisé en ce que l'on fait réagir sur les semences, les végétaux et/ou leur habitat une quantité régulatrice efficace d'une sulfonylurée de formule I de la revendication 1 ou de l'un de ses sels.

7. Procédé de préparation des sulfonylurées de formule I de la revendication 1 dans laquelle A a une signification autre que COOH, caractérisé en ce que l'on fait réagir un sulfonylisocyanate II

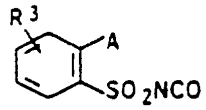

II

de manière connue en soi, dans un solvant organique inerte, avec une quantité voisine de la quantité stoechiométrique d'une 2-amino-4-fluoralcoxy-1,3,5-triazine substituée de formule III

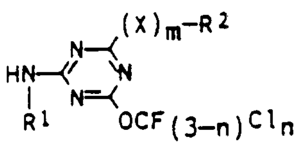

III

8. Procédé de préparation des sulfonylurées de formule I de la revendication 7, caractérisé en ce que l'on fait réagir un carbamate de formule IV

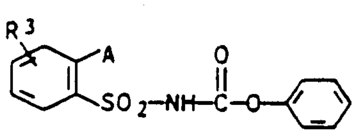

IV

de manière connue en soi, dans un solvant organique inerte, à une température de 0 à 120 °C, avec une quantité voisine de la quantité stoechiométrique d'une 2-amino-4-fluoralcoxy-1,3,5-triazine substituée III.

9. Procédé de préparation des sulfonylurées de formule I de la revendication 7, caractérisé en ce que l'on fait réagir un sulfonamide correspondant de formule V

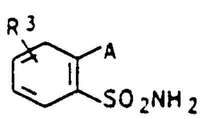

V

de manière connue en soi, dans un solvant organique inerte, avec un phénylcarbamate VI

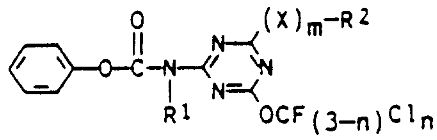

VI

**10.** 2-amino-4-fluoralcoxy-1,3,5-triazines substituées de formule générale IIIa

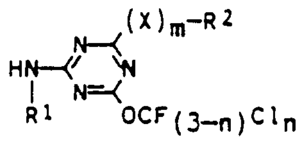

IIIa

dans laquelle m est égal à 1 et n à 0 ou 1, et les symboles ont les significations suivantes.
$R^1$
l'hydrogène, un groupe alkyle en C1-C4, alcényle en C3-C6 ou alcynyle en C3-C6 ;
$R^2$
un groupe alkyle en C1-C4, alcényle en C3-C6 ou alcynyle en C3-C6 ;
X
O, S ou N-4 dans lequel $R^4$ représente l'hydrogène ou un groupe alkyle en C1-C4.

**11.** Procédé de préparation des 2-amino-4-fluoralcoxy-1,3,5-triazines de formule générale IIIa de la revendication 10, caractérisé en ce que l'on fait réagir des 2-amino-4-fluoralcoxy-6-halogéno-1,3,5-triazines de formule IIIb

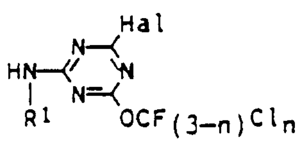

IIIb

dans laquelle Hal représente le fluor, le chlore ou le brome et $R^1$ et n ont les significations indiquées ci-dessus, avec un réactif nucléophile de formule XVI

$H\text{-}X\text{-}R^2$ XVI

dans laquelle X et $R^2$ ont les significations indiquées ci-dessus, ou l'un de ses sels.

**12.** 2-amino-4-méthoxy-6-trifluorométhoxy-1,3,5-triazine.

**13.** 2-amino-4-méthoxy-6-chlorodifluorométhoxy-1,3,5-triazine.

**14.** 2-amino-4-méthylamino-6-trifluorométhoxy-1,3,5-triazine.

**15.** 2-amino-4-méthylamino-6-chlorodifluorométhoxy-1,3,5-triazine.

**16.** 2-amino-4-diméthylamino-6-trifluorométhoxy-1,3,5-triazine.

**17.** 2-amino-4-chlorodifluorométhoxy-6-diméthylamino-1,3,5-triazine.

**18.** 2-amino-4-éthoxy-6-trifluorométhoxy-1,3,5-triazine.

**19.** 2-amino-4-chlorodifluorométhoxy-6-éthoxy-1,3,5-triazine.

**20.** 2,4-bisméthylamino-6-trifluorométhoxy-1,3,5-triazine.